# EUROPEAN PATENT APPLICATION

(11) **EP 4 850 635 A1**
(43) Date of publication of application: **09.12.2026**
(21) Application number: 25747640.8
(22) Date of filing: 21.01.2025
(51) Int. Cl.: A61F 2/24

(54) **DELIVERY SYSTEM FOR MEDICAL DEVICE**

(30) Priority: 30.01.2024 CN 202410126168; 22.07.2024 CN 202410985676
(71) Applicant: Hangzhou Valgen Medtech Co., Ltd., Hangzhou, Zhejiang 310051 (CN)
(72) Inventor: ZHU, Peng, Hangzhou, Zhejiang 310051 (CN); LI, Liguang, Hangzhou, Zhejiang 310051 (CN); ZHANG, Tingchao, Hangzhou, Zhejiang 310051 (CN); YUAN, Yongxin, Hangzhou, Zhejiang 310051 (CN)
(74) Representative: Mathys & Squire
(86) International application number: PCT/CN2025/073647
(87) International publication number: WO 2025/162068

(57) **Abstract**

The present disclosure provides a valve clip system including a first clip member, a second clip member, a catheter, a first actuation element, a second actuation element, and a handle. The first actuation element is connected to the first clip member and extends proximally through the catheter. The second actuation element is connected to the second clip member and extends proximally through the catheter. The handle includes a handle housing connected to a proximal end of the catheter, a first actuation controller and a second actuation controller respectively connected to the first actuation element and the second actuation element, a first linkage member and a second linkage member respectively coupled to the first actuation controller and the second actuation controller, and an unlocking member. The two linkage members cooperate with the unlocking member to complete linked control and independent control of the first actuation controller and the second actuation controller. The valve clip system can achieve simultaneous clamping of two leaflets of a heart valve, which not only ensures a symmetry of clamping and a consistency of clamping depth, but also ensures a therapeutic effect of surgery.

## Description

### CROSS-REFERENCE TO RELATED APPLICATIONS

This application claims priority to Chinese Patent Application No. 202410126168.0, filed with the China National Intellectual Property Administration on January 30, 2024, entitled "Valve Clip System with Linked Control," and Chinese Patent Application No. 202410985676.4, filed with the China National Intellectual Property Administration on July 22, 2024, entitled "Delivery System for Medical Device," the entire contents of which are incorporated herein by reference.

### TECHNICAL FIELD

The present disclosure relates to technical field of medical devices, and in particularly, to a delivery system for a medical device.

### BACKGROUND

Heart disease is one of the major health threats worldwide, among which heart valve disease is the most common. For heart valve diseases caused by various pathological changes, compared with open-heart surgery, interventional surgery has the characteristics of less invasiveness, minimal invasiveness, and safety. Therefore, in recent years, the technology for treating pathological sites through interventional surgery has developed rapidly. Currently, mainstream interventional surgical solutions include artificial chordae implantation, edge-to-edge repair, annuloplasty, and the like. Generally, this type of surgeries are performed in a minimally invasive manner via catheter-based interventional technology introduced into the patient's body, and need to be completed under the guidance of medical imaging with remote control outside the patient's body. Since interventional surgery is performed under non-direct vision conditions, how to use the delivery system to deliver the interventional device to the target treatment site in the patient's body and correctly position the interventional device to successfully perform the surgery still brings various challenges, such as easily causing low surgical success rates, and even endangering the patient's life safety in severe cases.

### SUMMARY

To address the deficiencies of the prior art, in a first aspect, the present disclosure provides a valve clip system, including:
a valve clip, including a jaw arm, a first clip member and a second clip member, where the first clip member and the second clip member are movable relative to the jaw arm;
a catheter;
a first actuation element and a second actuation element, where the first actuation element is releasably connected to the first clip member, the first actuation element extends proximally through the catheter and is axially movable within the catheter; the second actuation element is releasably connected to the second clip member, the second actuation element extends proximally through the catheter and is axially movable within the catheter; and
a handle, the handle including:
   a handle housing, the handle housing being connected to a proximal end of the catheter;
   a first actuation controller and a second actuation controller, the first actuation controller being connected to a proximal end of the first actuation element, the second actuation controller being connected to a proximal end of the second actuation element;
   a first linkage member and a second linkage member, the first linkage member being coupled to the first actuation controller, the second linkage member being coupled to the second actuation controller; and
   an unlocking member, where the unlocking member is connected to the first linkage member, the unlocking member is shiftable relative to the second linkage member under an external force to switch between the first actuation controller and the second actuation controller are coupled to each other, andthe coupling therebetween is released, thereby controlling the first actuation element and the second actuation element to move synchronously or independently.

In a second aspect, the present disclosure provides a valve clip system, including:
a valve clip, including a jaw arm, and a first clip member and a second clip member, where the first clip member and the second clip member are movable relative to the jaw arm;
a catheter;
a first actuation element and a second actuation element, where the first actuation element is releasably connected to the first clip member, the first actuation element extends proximally through the catheter and is axially movable within the catheter; the second actuation element is releasably connected to the second clip member, the second actuation element extends proximally through the catheter and is axially movable within the catheter; and
a handle, the handle including:
   a handle housing, the handle housing being connected to a proximal end of the catheter;
   a first actuation controller and a second actuation controller, the first actuation controller being connected to a proximal end of the first actuation element, the second actuation controller being connected to a proximal end of the second actuation element; and
   a first linkage member and a second linkage member, where the first linkage member is coupled to the first actuation controller, the second linkage member is coupled to the second actuation controller; the first linkage member and the second linkage member are synchronously rotatable relative to the handle housing, so as to synchronously control the first actuation controller and the second actuation controller to move proximally or distally, thereby synchronously driving the first actuation element and the second actuation element to move proximally or distally.

In a third aspect, the present disclosure provides a valve clip system with linked control, including:
a valve clip, including a jaw arm, a first clip member and a second clip member, where the first clip member and the second clip member are movable relative to the jaw arm;
a catheter;
a first actuation element and a second actuation element, where the first actuation element is releasably connected to the first clip member, the first actuation element extends proximally through the catheter and is axially movable relative to the catheter; the second actuation element is releasably connected to the second clip member, the second actuation element extends proximally through the catheter and is axially movable relative to the catheter; and
a handle, the handle including:
   a handle housing, the handle housing being connected to a proximal end of the catheter and having a central axis axially aligned with the catheter;
   a first actuation controller and a second actuation controller, the first actuation controller being connected to a proximal end of the first actuation element, the second actuation controller being connected to a proximal end of the second actuation element; and
   a linkage member, the linkage member connecting the first actuation controller and the second actuation controller;
   where the first actuation controller has a first axis intersecting the central axis, the second actuation controller has a second axis intersecting the central axis; the linkage member is configured to synchronously control the first actuation controller to move relative to the handle housing along the first axis, and the second actuation controller to move relative to the handle housing along the second axis.

In a fourth aspect, the present disclosure provides a delivery system for a medical device, including:
a first delivery device, including a first handle and a first catheter extending distally from the first handle, the first catheter including a bendable distal section; and
a second delivery device, including a second handle and a second catheter extending distally from the second handle, where the second catheter coaxially extends through the first delivery device, the second catheter along its axial length has a first section and a second section with a reduced outer diameter, the second section is located distal to the first section;
where the first handle is manipulated to force the distal section of the first catheter to bend, the second section of the second catheter can be shapeable into a curve configuration by the bent distal section, and can at least partially recover from the curve configuration to an initial configuration after extending out of the bent distal section.

In a fifth aspect, the present disclosure provides a transcatheter delivery system for a medical device, including:
a first delivery device, including a first handle and a first catheter extending distally from the first handle, the first catheter including a bendable and/or pre-formed distal section; and
a second delivery device, including a second handle and a second catheter extending distally from the second handle, the second catheter extending coaxially through the first delivery device; and
a third delivery device, including a third handle and a third catheter extending distally from the third handle, the third catheter including a bendable distal section, the first catheter extending coaxially through the third delivery device;
where the second catheter includes a proximal section extending along its axial length, the proximal section includes a laser-cut tube; the second delivery device is configured to deliver the medical device via a blood vessel to a target site of the heart under a bending cooperation of the distal section and the bendable section, and the proximal section is always positioned outside a body.

In a sixth aspect, the present disclosure provides a delivery system for a medical device, including:
a first delivery device, including a first handle and a first catheter extending distally from the first handle, where a distal coupler is fixedly disposed at a distal end of the first catheter; and
a second delivery device, including a second handle and a second catheter extending distally from the second handle, where the second catheter coaxially extends through the first delivery device; a protective coupler is fixedly disposed at a distal end of the second catheter, the medical device is releasably connected to the protective coupler;
where the distal coupler has an axially through engagement cavity, an outer surface of the protective coupler forms a circumferential engagement surface, the circumferential engagement surface is configured for circumferentially engaging within the engagement cavity, to shorten an axial distance of the medical device relative to the distal coupler.

The present disclosure improves surgical success rates by rationally and effectively designing the valve clip system and delivery system to successfully deliver the medical device to a target site in the patient's body.

### BRIEF DESCRIPTION OF THE ACCOMPANYING DRAWINGS

The drawings herein are incorporated into the specification and constitute a part of this specification, illustrating embodiments consistent with the present disclosure, and are used together with the specification to explain the principles of the present disclosure.

In order to more clearly illustrate the technical solutions of the embodiments of the present disclosure, the drawings needed in the embodiments will be briefly introduced below. Obviously, the drawings in the following description are merely some embodiments provided by the present disclosure, and those of ordinary skill in the art can obtain other drawings based on these drawings without creative efforts.
FIG. 1 shows a structural view of a delivery system for a medical device in accordance with a first embodiment, with a second section of a second catheter shapeable into a curved configuration.
FIG. 2 shows a state view of the second section in FIG. 1 recovering to an initial configuration.
FIG. 3 shows a cross-sectional view of a coil extending axially through the second catheter.
FIG. 4 shows a state view of the second catheter extending out of a distal end of the first catheter and moving a stroke distance.
FIG. 5 shows a structural view of the second catheter extending coaxially through the first catheter.
FIG. 6 shows a structural view of a lubricating coating on the second catheter.
FIG. 7 shows a structural view of the delivery system in FIG. 1 further including a third delivery device.
FIG. 8 shows another structural view of a lubricating coating on the second catheter.
FIG. 9 shows a schematic view of a bending angle range of a distal section of the first catheter.
FIG. 10 shows a structural view of a traction member of the first catheter.
FIG. 11 shows a schematic view of the first catheter extending out of the third catheter.
FIG. 12 shows a schematic view of a bending angle range of the first catheter under an action of a traction member and a pre-formed curve.
FIG. 13 shows a structural view of a transcatheter delivery system for a medical device in accordance with a second embodiment.
FIG. 14 shows a structural view of a second catheter in FIG. 13.
FIG. 15 shows a structural view of a laser-cut tube in FIG. 14.
FIG. 16 respectively shows layered structures of the second catheter in a proximal section, a middle section, and a distal section.
FIG. 17 shows a schematic view of a delivery system for a medical device in accordance with a third embodiment.
FIG. 18 shows a structural view of cooperation between a distal coupler and a protective coupler in FIG. 17.
FIGS. 19-20 respectively show side cross-sectional views of the first delivery device and the second delivery device in FIG. 18.
FIG. 21 shows a state view of a valve clip system in accordance with a fourth embodiment in a linked control state, with a valve clip in a state where a first clip member and a second clip member are closed relative to a jaw arm.
FIG. 22 shows a state view of the first clip member and the second clip member in FIG. 21 being opened relative to the jaw arm under linked control.
FIG. 23 shows a structural view of the valve clip system in FIG. 22 after an upper housing is covered.
FIG. 24 shows a structural view of the handle in FIG. 22.
FIG. 25 shows a schematic view of a sealing design of the handle in FIG. 24.
FIG. 26 shows a structural view of a first control handle in FIG. 25.
FIG. 27 shows a perspective schematic view of a first manner of releasing linked control.
FIG. 28 shows a structural view of a second manner of releasing linked control.
FIG. 29 shows a structural view of a state in FIG. 28 with the upper housing removed.
FIG. 30 shows a perspective cross-sectional view of an unlocking member in FIG. 28 being pulled up to release linked control.
FIG. 31 shows a structural view of the unlocking member in FIG. 30.
FIG. 32 shows a structural view of a knob in FIG. 31.
FIG. 33 shows a structural view of the upper housing in FIG. 31.
FIG. 34 shows a structural view of a connecting rod in FIG. 31.
FIG. 35 shows a perspective cross-sectional view of the unlocking member in FIG. 30 connected to the upper housing.
FIG. 36 shows a side cross-sectional view of FIG. 35.
FIG. 37 shows another structural view of the first control handle.
FIG. 38 shows a state view of a valve clip system in accordance with a fifth embodiment in a linked control state, with a valve clip in a state where a first clip member and a second clip member are closed relative to a jaw arm.
FIG. 39 shows a state view of the first clip member and the second clip member in FIG. 38 being opened relative to the jaw arm under linked control.
FIG. 40 shows a structural view of the handle in accordance with the fifth embodiment.
FIG. 41 shows a structural view of a first actuation controller and a second actuation controller in FIG. 40.
FIG. 42 shows another structural view of the first actuation controller and the second actuation controller in FIG. 40.

### DETAILED DESCRIPTION OF ILLUSTRATED EMBODIMENTS

In order to make the technical problems, technical solutions, and technical effects addressed by the embodiments of the present disclosure clearer, the technical solutions of the present disclosure will be described clearly and completely below in conjunction with the drawings in the embodiments of the present disclosure. Obviously, the described embodiments are merely a part of the embodiments of the present disclosure, rather than all embodiments. Based on the embodiments in the present disclosure, all other embodiments obtained by those of ordinary skill in the art without creative efforts shall fall within the protection scope of the present disclosure.

In the description of the present disclosure, it should be noted that, in the field of interventional medical devices, the proximal end refers to an end closer to the operator, and the distal end refers to an end farther from the operator; the axial direction refers to the direction parallel to a line connecting the distal center and the proximal center of the medical device; the radial direction refers to a direction perpendicular or substantially perpendicular to the axial direction; the circumferential direction refers to a direction surrounding the axial direction. It is worth noting that regardless of the "end" appearing in terms such as "proximal end," "distal end," "one end," "the other end," "first end," "second end," "initial end," "terminal end," "both ends," "free end," "upper end," "lower end," etc., it is not limited to the end head, end point, or end surface, but also includes a portion extending a certain axial distance and/or radial distance from the end head, end point, or end surface on the element to which the end head, end point, or end surface belongs. The above definitions are merely for convenience of expression and should not be construed as limitations on the present disclosure.

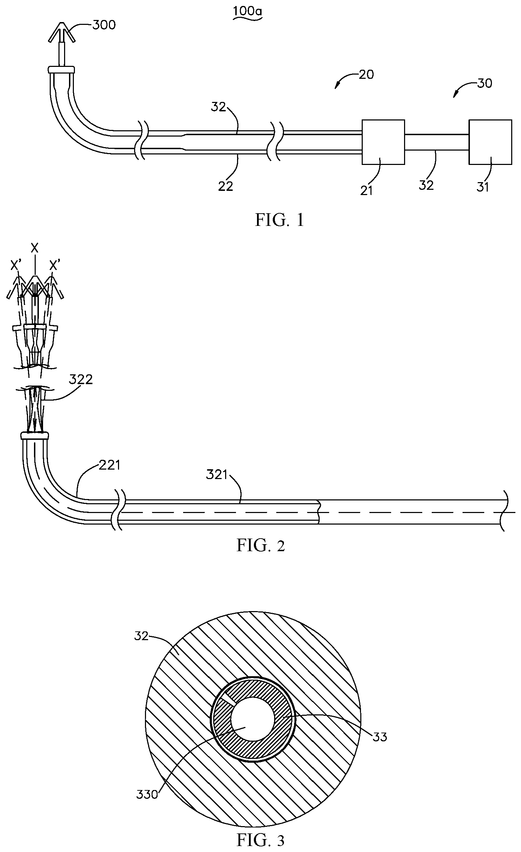

In the first embodiment, the present disclosure provides a delivery system 100a for a medical device, for delivering a medical device 300 into a patient's body to treat heart disease. For example, the delivery system 100a can enter the patient's body via a transapical approach or via a transcatheter approach. Specifically, please refer to FIGS. 1 and 2, the delivery system 100a includes a first delivery device 20 and a second delivery device 30. The first delivery device 20 includes a first handle 21 and a first catheter 22 extending distally from the first handle 21. The first catheter 22 includes a bendable distal section 221. The second delivery device 30 includes a second handle 31 and a second catheter 32 extending distally from the second handle 31. The second catheter 32 extends coaxially through the first delivery device 20.

The second catheter 32 has a first section 321 and a second section 322 with a reduced outer diameter, where the first section 321 and the second section 322 are distributed along an axial length of the second catheter 32. The second section 322 is located distal to the first section 321. As shown in FIG. 1, the first handle 21 is manipulated to force the distal section 221 of the first catheter 22 to bend, the second section 322 of the second catheter 32 is shapeable into a curve configuration by the bent distal section 221, and is at least partially recovered from the curve configuration shown in FIG. 1 to an initial configuration shown in FIG. 2 after extending out of the bent distal section 221. In at least one embodiment, the initial configuration is an unbent configuration.

For some applications, application scenarios in which the second section 322 of the second catheter 32 is shapeable into the curve configuration include: first manipulating the first handle 21 to force the distal section 221 of the first catheter 22 to bend, and then driving the second catheter 32 to translate axially to control its second section 322 to pass through the bent distal section 221 and be shapeable into the curve configuration; or, the second section 322 of the second catheter 32 is pre-accommodated within the distal section 221 of the first catheter 22, and when the first handle 21 is manipulated to force the distal section 221 to bend, the second section 322 is shapeable into the curve configuration by the bent distal section 221.

Because the outer diameter of the second section 322 of the second catheter 32 is reduced relative to the first section 321 of the second catheter 32, the recovery performance of the second section 322 is necessarily stronger than that of the first section 321. Therefore, after the second section 322 extends out of the bent distal section 221, the second section 322 can necessarily rapidly recover from the curve configuration to the initial configuration, thereby ensuring that during heart surgery, after the second catheter 32 extends out of the first catheter 22, the second catheter 32 can continuously maintain substantial coaxiality with the first catheter 22, especially substantial coaxiality of their distal ends, such as continuously maintaining X-axis coaxiality, ultimately ensuring the precision of subsequent manipulation of the second catheter 32 relative to the first catheter 22, greatly improving surgical precision.

The delivery system 100a of the present disclosure ensures the recovery performance of the second catheter 32, avoiding the deficiency in the prior art that after the second catheter 32 extends out of the first catheter 22, it is highly prone to unexpected movement or bending deformation, ultimately resulting in the inability of the second catheter 32 to continuously maintain coaxiality with the first catheter 22, such as causing the X'-axis of the distal second catheter 32 to deviate from the X-axis of the first catheter 22 at the distal end(see FIG. 2). Meanwhile, the delivery system 100a of the present disclosure further ensures the precision of manipulation during surgery, greatly shortening surgical time, avoiding the excessive surgical operations required in the prior art to re-orient the first catheter 22 due to the second catheter 32 being highly prone to unexpected deformation after extending out of the first catheter 22, which easily leads to various other unexpected situations and prolonged surgical time.

In addition, under the manipulation of the second handle 31, the second catheter 32 can move within the first catheter 22, such as rotating and/or translating axially. During rotation and/or axial translation, especially when the second catheter 32 moves within the already bent distal section 221, excessive friction is more likely to be generated between the second catheter 32 and the distal section 221, thereby causing unsmooth rotation and/or axial translation. In the delivery system 100a of the present disclosure, the second section 322 of the second catheter 32 has a reduced outer diameter relative to the first section 321, thereby greatly reducing the friction between the second section 322 and the distal section 221 when the second section 322 passes through the bent distal section 221, thereby ensuring that the second section 322 of the second catheter 32 can smoothly extend out of the distal section 221 of the second catheter 32, further improving the delivery effect of the delivery system 100a.

Of course, in order to enhance the recovery performance of the second catheter 32 in the delivery system 100a, in further embodiments, as shown in FIG. 3, the second delivery device 30 further includes an axially incompressible coil 33, and the coil 33 extends coaxially through the second catheter 32. The coil 33 can enhance the recovery performance of the second catheter 32, i.e., the coil 33 enhances the recovery performance of the second section 322 recovering from the curve configuration to the initial configuration. Preferably, the coil 33 is a spring tube coiled with flat wire.

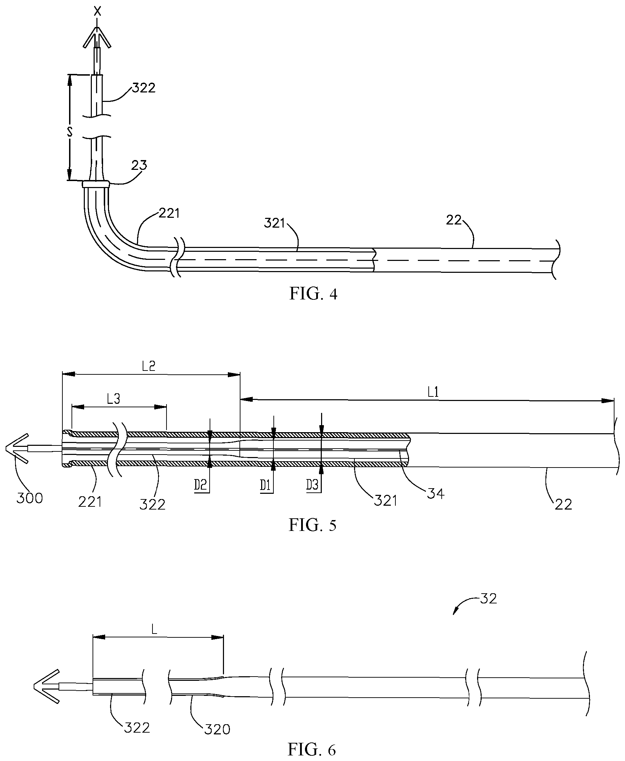

For some applications, the coil 33 extends coaxially through an entire inner cavity of the second catheter 32; that is, the axial length of the coil 33 is equal to the axial length of the second catheter 32. For other applications, in order to ensure that a release rod 34 in FIG. 5 can smoothly pass through and move within the second catheter 32, the coil 33 forms an axially extending through cavity 330 for the release rod 34 to pass through.

In at least one embodiment, as shown in FIG. 4, the second catheter 32 can extend out of the distal end of the distal section 221 of the first catheter 22 and can move a stroke distance S. The axial length L2 of the second section 322 of the second catheter 32 (see FIG. 5) is equal to the stroke distance S. After moving the stroke distance S, the second section 322 of the second catheter 32 will completely extend out of the bent distal section 221, and the distal-most end of the first section 321 will be positioned at the distal-most end of the distal section 221. In view of the gap between the second section 322 and the first catheter 22 being larger than the gap between the first section 321 and the first catheter 22, positioning the distal-most end of the first section 321 at the distal-most end of the distal section 221 is more conducive to coaxial positioning between the first catheter 22 and the second catheter 32, such as maintaining X-axis coaxial positioning. Therefore, the above design enhances the coaxial positioning of the first catheter 22 and the second catheter 32 without needing to re-orient the first catheter 22, thereby providing precise positioning for subsequent rotation and other operations of the second catheter 32. Preferably, the stroke distance S is in a range of 40 mm to 80 mm.

For some applications, a distal coupler 23 is fixed to the distal end of the distal section 221 of the first catheter 22. The second catheter 32 can sequentially pass through the first catheter 22 and the distal coupler 23, and then move the stroke distance S. After moving the stroke distance S, the distal-most end of the first section 321 will be positioned at the distal coupler 23. The distal coupler 23 is made of a metal material, such as stainless steel. In view of the smoothness and low friction of metal materials, the metal distal coupler 23 is more conducive to guide the first catheter 22 to advance smoothly within blood vessels or other devices.

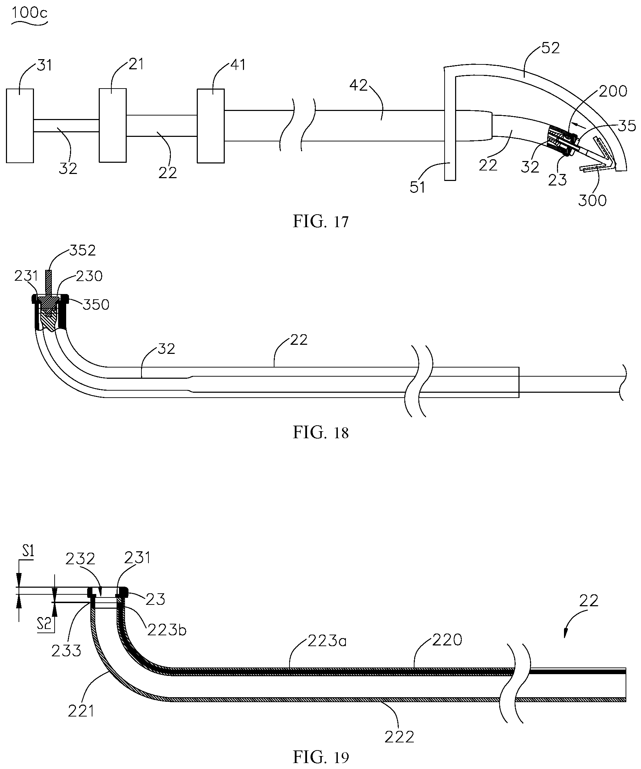

For other applications, a protective coupler 35 is fixed to at the distal end of the second section 322 of the second catheter 32. Specifically, the distal coupler 23 defines an axially through engagement cavity 230, an outer surface of the protective coupler 35 forms a circumferential engagement surface 350, and the circumferential engagement surface 350 can circumferentially engage within the engagement cavity 230. For the specific structures, cooperation relationships, and corresponding technical effects of the distal coupler 23 and the protective coupler 35, please refer to the subsequent discussion of FIGS. 17-20, which will not be specifically repeated here.

In at least one embodiment, as shown in FIG. 5, the outer diameter difference between the first section 321 and the second section 322 of the second catheter 32 is in the range of 0.3 mm to 1.05 mm. Specifically, assuming an axial length of the first section 321 is L1, an outer diameter of the first section 321 is D1, an axial length of the second section 322 is L2, an outer diameter of the second section 322 is D2, an inner diameter of the first catheter 22 is D3, and an axial length of the distal section 221 of the first catheter 22 is L3. In order to ensure that the second catheter 32 can extend through the first catheter 22 and can move smoothly within the first catheter 22 to reduce mutual interference between the two during delivery, necessarily, D3 > D1 > D2. Meanwhile, in order to allow the axial length L2 of the second section 322 with reduced outer diameter to completely cover within the bendable distal section 221, to reduce friction generated by the second catheter 32 moving within the already bent distal section 221, preferably, L1 > L2 > L3. For some applications, the outer diameter D1 of the first section 321 is in the range of 3.65 mm to 3.95 mm, the outer diameter D2 of the second section 322 is in the range of 2.9 mm to 3.65 mm, and the inner diameter D3 of the first catheter 22 is in the range of 4.1 mm to 4.8 mm.

Further, in order to avoid a risk of fracture easily caused by an abrupt change in outer diameter at a junction between the first section 321 and the second section 322 of the second catheter 32, a smooth taper with a taper distance in the range of 10 mm to 25 mm is disposed between the first section 321 and the second section 322 to achieve a smooth transition between the first section 321 and the second section 322.

It should be specially noted that, in at least one embodiment, please continue to refer to FIG. 5, the medical device 300 includes a valve clip, and the second delivery device 30 uses a release rod 34 movable within the second catheter 32 to releasably engage the valve clip to the distal end of the second catheter 32 or the protective coupler 35 via the release rod 34. In order to ensure that the release rod 34 can move smoothly within the second catheter 32, the inner diameter of the first section 321 is equal to the inner diameter of the second section 322.

Of course, in order to further reduce friction between the first catheter 22 and the second catheter 32, as shown in FIGS. 6-8, the second catheter 32 further includes a lubricating coating 320. The lubricating coating 320 can be made of biocompatible polyvinylpyrrolidone (PVP) material, attached to an outer surface of the second catheter 32 through a photocuring process, or can be a PTFE inner etched tube with a low friction coefficient, attached to the outer surface of the second catheter 32 through a heat fusion welding process.

For some applications, as shown in FIG. 6, the lubricating coating 320 is disposed on the outer surface of the second section 322 of the second catheter 32, and a length L of the lubricating coating 320 is equal to an axial length L2 of the second section 322.

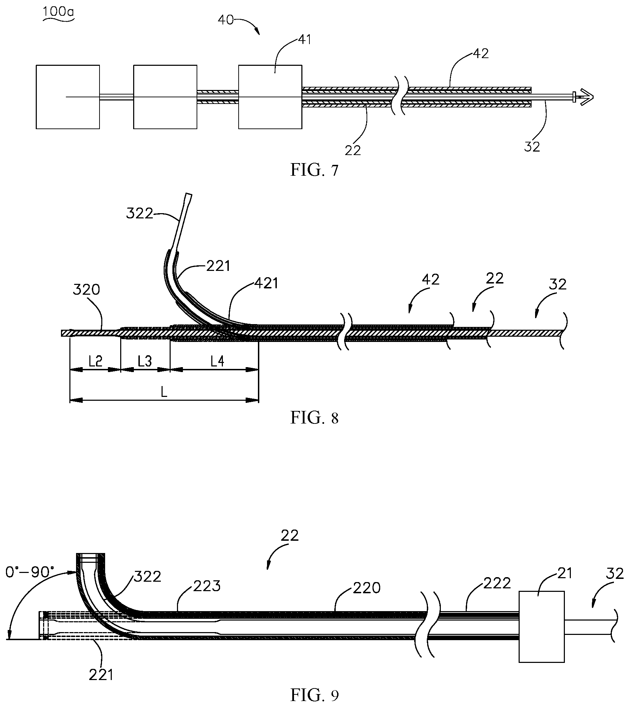

For other applications, as shown in FIGS. 7-8, the delivery system 100a further includes a third delivery device 40; the third delivery device 40 includes a third handle 41 and a third catheter 42 extending distally from the third handle 41. The first catheter 22 extends coaxially through the third delivery device 40, and can move within the third delivery device 40, such as by twisting and/or axial translation. The third catheter 42 includes a bendable segment 421 at a distal end, preferably, a bending angle range of the bendable segment 421 is 0 degree to 120 degrees. Further, an outer surface of the second section 322 and at least part of the first section 321 of the second catheter 32 has a continuous lubricating coating 320, and a length L of the lubricating coating 320 is greater than or equal to a sum of the axial length L2 of the second section 322, the axial length L3 of the distal section 221 of the first catheter 22, and the axial length L4 of the bendable segment 421 of the third catheter 42. That is, the length L of the lubricating coating 320 ≥ L2 + L3 + L4, and the preferred distance range of L is between 120 mm and 180 mm. Preferably, the length L of the lubricating coating 320 is equal to the axial length of the second catheter 32, that is, L = L1 + L2, and an entire outer surface of the second catheter 32 is coated with the lubricating coating 320.

Further, in order to ensure that the distal section 221 of the first catheter 22 can move within a preset bending angle range, so that the first catheter 22 can be positioned at a target site in the heart according to a preset route, such as positioned substantially at the center above the annulus, in at least one embodiment, as shown in FIG. 9, the first catheter 22 further includes a proximal section 222 and a traction lumen 220 at least partially extending through the proximal section 222 and the distal section 221. The distal end of at least one traction member 223 is attached to the distal section 221, and the proximal end of the traction member 223 extends through the traction lumen 220 and is attached to the first handle 21. The first handle 21 is manipulated to actuate the traction member 223 to pull the distal section 221, so that the distal section 221 moves within a bending angle range of 0 degree to 90 degrees, thereby shaping the second section 322 of the second catheter 32 into the curve configuration having substantially the same curvature as the distal section 221.

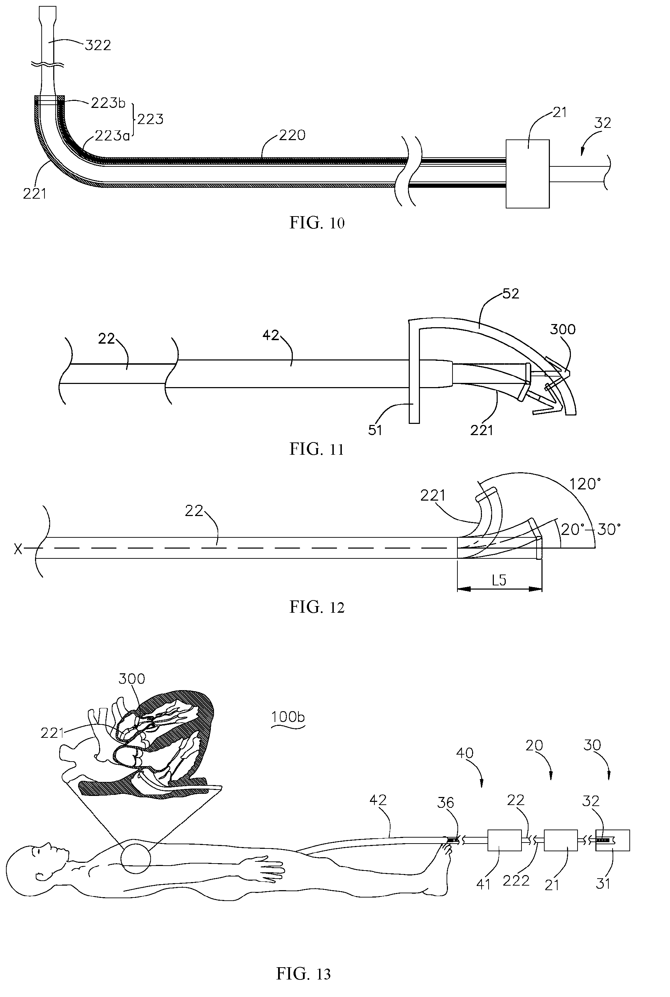

Specifically, please further refer to FIG. 10, the traction member 223 includes a traction wire 223a and a distal pull ring 223b. The distal pull ring 223b is fixed to the distal section 221, such as heat-fused to the distal end of the distal section 221, the distal end of the traction wire 223a is connected to the distal pull ring 223b, and the proximal end of the traction wire 223a extends through the traction lumen 220 and is attached to the first handle 21. Under the manipulation of the first handle 21, the traction wire 223a can be pulled and tensioned to force the distal section 221 of the first catheter 22 to bend or deform. The second section 322 shapeable into the curve configuration by the bent distal section 221 can extend out of the distal section 221 of the first catheter 22, and under the recovery performance of the second section 322, the second section 322 can quickly recover from the curve configuration to the initial configuration, or at least partially recovered.

In view of the application scenario shown in FIG. 11, the third catheter 42 passes through the atrial septum 51 of the heart and is positioned in the atrium. Then, the first catheter 22 is manipulated to extend out of the distal end of the third catheter 42, and then the first catheter 22 is manipulated to bend. However, during the process of manipulating the first catheter 22 to extend out of the distal end of the third catheter 42, the first catheter 22 or the medical device 300 extending out of the distal end of the first catheter 22 is highly likely to accidentally touch the inner side of the atrial wall 52 opposite to the atrial septum 51, thereby causing the defect of atrial tissue damage.

In order to ensure that the delivery system 100a can completely avoid the first catheter 22 touching heart tissue when extending out of the distal end of the third catheter 42 in the application scenario shown in FIG. 11, such as keeping away from the inner side of the atrial wall 52, further, please refer to FIGS. 11 and 12 simultaneously, the distal section 221 of the first catheter 22 has a pre-formed curve. Preferably, the angle range of the pre-formed curve is between 20 degrees and 30 degrees, and the axial length L5 of that is in the range of 20 mm to 25 mm. That is, when the first catheter 22 is in a natural state without external force, its distal section 221 bends outward at an angle range deviating from the X-axis by 20 degrees to 30 degrees.

In view of the distal section 221 of the first catheter 22 being configured to move within a bending angle range of 0 degree to 90 degrees under the traction of the traction member 223, therefore, under the combined action of the pre-formed curve and the traction member 223, the bending angle range of the distal section 221 of the first catheter 22 is superimposed, that is, the distal section 221 of the first catheter 22 can move within a bending angle range of 20 degrees to 120 degrees, thereby shaping the second section 322 of the second catheter 32 into a curve configuration having substantially the same curvature as the distal section 221 of the first catheter 22.

Generally, when the delivery system enters the interior of the heart via a transcatheter approach, in order to guide the distal end of the catheter, such as the distal end of the first catheter 22, the distal end of the second catheter 32, and/or the distal end of the third catheter 42, to a target site inside the heart, the physician must apply axial driving force and/or torsional force from the proximal end of the catheter. The catheter for transmitting these forces from the proximal end to the distal end must be stiff enough to be pushed through blood vessels, but also flexible enough to pass through tortuous blood vessels, and also requires sufficient torsional stiffness to transmit the applied torque. Thus, the designer needs to strike a balance between achieving axial stiffness, torsional stiffness, and flexibility.

In the second embodiment, as shown in FIG. 13, the transcatheter delivery system 100b for the medical device 300 enters the human body via a transcatheter approach, such as via the femoral vein into the human body, for delivering the medical device 300 into the heart in the body to treat heart disease.

Specifically, the transcatheter delivery system 100b includes a first delivery device 20, a second delivery device 30, and a third delivery device 40. The first delivery device 20 includes a first handle 21 and a first catheter 22 extending distally from the first handle 21. The first catheter 22 includes a bendable and/or pre-formed distal section 221. The second delivery device 30 includes a second handle 31 and a second catheter 32 extending distally from the second handle 31. The second catheter 32 extends coaxially through the first delivery device 20. The third delivery device 40 includes a third handle 41 and a third catheter 42 extending distally from the third handle 41. The third catheter 42 includes a bendable segment 421 at a distal end (see FIG. 8). The first catheter 22 extends coaxially through the third delivery device 40.

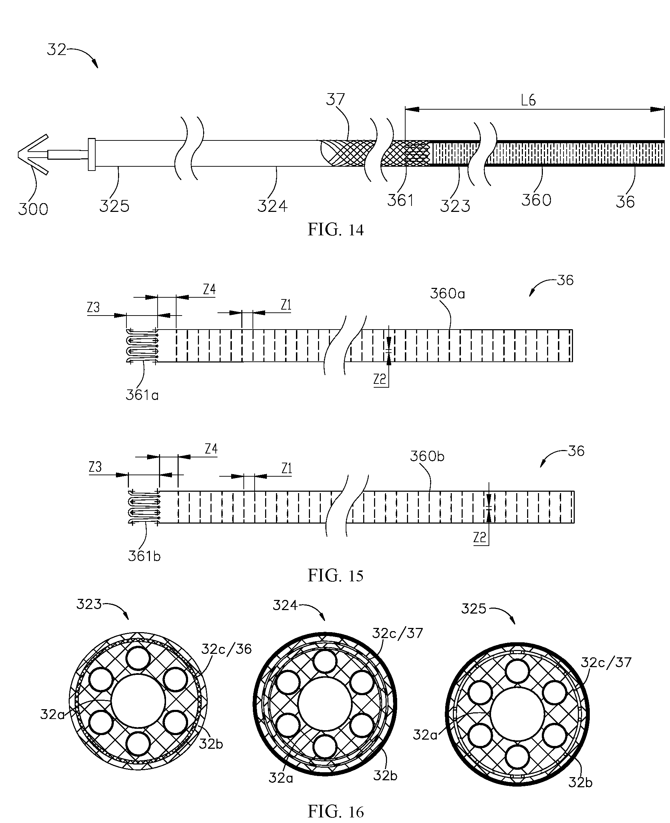

Further, the second catheter 32 includes different sections along its axial length to balance the needs for axial stiffness, torsional stiffness, and flexibility. Specifically, as shown in FIG. 14, the second catheter 32 includes a proximal section 323 extending along its axial length. In order to ensure that its proximal section 323 can simultaneously meet the requirements of twistability and support stability, the proximal section 323 includes a laser-cut tube 36. The second delivery device 30 can deliver the medical device 300 via a blood vessel to a target site of the heart under the bending cooperation of the distal section 221 and the bendable segment 421, while the proximal section 323 will always be positioned outside the body. That is, the laser-cut tube 36 of the proximal section 323 will not enter the blood vessel, neither affecting the compliance requirements of the blood vessel nor creating the risk of puncturing the blood vessel, ensuring the safety of the surgery.

It can be understood that, in order to balance the needs of the transcatheter delivery system 100b for axial stiffness, torsional stiffness, and flexibility, the second catheter 32 is designed as a segmented structure. Specifically, please continue to refer to FIG. 14, the second catheter 32 further includes a middle section 324 and a distal section 325 extending along its axial length, and the middle section 324 connects the distal section 325 and the proximal section 323. The distal section 325 can be either a distal section with no change in outer diameter as shown in FIG. 14, or a distal section with a changed outer diameter as shown in FIG. 2. In FIG. 2, the axial length of the distal section 325 is greater than the axial length of the second section 322, that is, the distal section 325 includes the second section 322 and part of the first section 321. Preferably, the length of the proximal section 323 is in the range of 400 mm to 600 mm, the length of the middle section 324 is in the range of 640 mm to 790 mm, and the length of the distal section 325 is in the range of 110 mm to 160 mm.

Please refer to FIGS. 14 and 15 simultaneously. In order to ensure the uniformity of support along the axial length of the proximal section 323 to avoid the risk of fracture, in at least one embodiment, the laser-cut tube 36 has a constant stiffness along its axial length, and its axial length range L6 is 400 mm to 600 mm (see FIG. 14), and its thickness range is 0.1 mm to 0.3 mm. Specifically, the laser-cut tube 36 has a plurality of cutting patterns 360, and the plurality of cutting patterns 360 are uniformly spaced around the circumference of the laser-cut tube 36. Multiple groups of cutting patterns 360 are uniformly arranged axially on the laser-cut tube 36, and each two adjacent groups of cutting patterns 360 are radially offset from each other or circumferentially offset from each other.

In at least one embodiment, in order to ensure uniform symmetry of the laser-cut tube 36 in both axial and radial directions, the cutting patterns 360 are one of symmetric waist-shaped patterns 360a, olive-shaped patterns 360b, elliptical patterns (not shown), and square patterns (not shown). Specifically, as shown in FIG. 15, the aspect ratio of each cutting pattern 360 (including waist-shaped pattern 360a and olive-shaped pattern 360b) is 20, and the axial spacing Z1 between two adjacent cutting patterns 360 is greater than the radial spacing or the circumferential spacing Z2. For example, Z1 can be 3.5 mm, and the range of Z2 can be 0.9 mm to 1.0 mm.

In addition, the distal-most end of the laser-cut tube 36 forms a fusion end 361 with openings (including fusion ends 361a and 361b) for connecting with a component in the middle section 324, such as fusing with a braided mesh in the middle section 324. The axial length Z3 of the fusion end 361 is 13 mm, and the axial spacing Z4 from the fusion end 361 to the distal-most cutting pattern 360 is 6 mm.

It can be understood that, in order to further balance the needs of the transcatheter delivery system 100b for axial stiffness, torsional stiffness, and flexibility, in at least one embodiment, the second catheter 32 is designed as a layered structure. Specifically, please refer to FIGS. 14 and 16 simultaneously. The tube wall of the second catheter 32 radially includes a first polymer layer 32a, a first reinforcement layer 32c, and a second polymer layer 32b from inside to outside, and the first polymer layer 32a and the second polymer layer 32b configured to bond to each other to encapsulate the first reinforcement layer 32c between the first polymer layer 32a and the second polymer layer 32b. The first reinforcement layer 32c includes the laser-cut tube 36 and a braided mesh 37 connected to the distal end of the laser-cut tube 36. Preferably, the laser-cut tube 36 is located in the proximal section 323, and the braided mesh 37 is located in the middle section 324 and the distal section 325.

Of course, in order to ensure the support of the proximal end of the first catheter 22, please refer to FIG. 13 again, the first catheter 22 further includes a proximal section 222 connected to the proximal end of the distal section 221, and the proximal section 222 includes a harder material than the distal section 221. After the transcatheter delivery system 100b delivers the medical device 300 via a blood vessel to a target site of the heart, part of the proximal section 222 is positioned outside the body, and another part is positioned inside the body. The harder material does not include a laser-cut tube, to avoid the proximal section 222 located inside the body from puncturing blood vessels or other contacted tissues.

Similarly, in order to ensure that the distal section 221 of the first catheter 22 can be positioned at a target site in the heart according to a preset route, while avoiding damage to heart tissue caused by the first catheter 22 touching heart tissue when extending out of the third catheter 42, further, please refer to FIGS. 9-12 again, the first catheter 22 further includes a traction lumen 220 at least partially extending through the proximal section 222 and the distal section 221. The distal end of at least one traction member 223 is attached to the distal section 221, and the proximal end of the traction member 223 extends through the traction lumen 220 and is attached to the first handle 21. Meanwhile, the distal section 221 of the first catheter 22 has a pre-formed curve, and the distal section 221 can move within a bending angle range of 20 degrees to 120 degrees under the combined action of the pre-formed curve and the traction member 223.

Of course, in other embodiments, the first catheter 22 and the third catheter 42 can also be designed as multi-segment layered structures, for which specific reference can be made to the detailed discussion of the second catheter 32, which will not be repeated here.

In the third embodiment, in order to avoid the defect that in the application scenario shown in FIG. 17, the axial distance between the medical device 300 and the distal end of the first catheter 22 is too large, resulting in the distal end of the medical device 300 being highly likely to accidentally touch the inner side of the atrial wall 52 opposite to the atrial septum 51, thereby causing atrial tissue damage, the present disclosure also provides a delivery system 100c for the medical device 300.

Specifically, please refer to FIGS. 17-20, the delivery system 100c includes a first delivery device 20 and a second delivery device 30. The first delivery device 20 includes a first handle 21 and a first catheter 22 extending distally from the first handle 21, and a distal coupler 23 is fixedly disposed at a distal end of the first catheter 22. The second delivery device 30 includes a second handle 31 and a second catheter 32 extending distally from the second handle 31, and the second catheter 32 extends coaxially through the first delivery device 20. A protective coupler 35 is fixedly disposed at a distal end of the second catheter 32, and the medical device 300 is releasably connected to the protective coupler 35. The distal coupler 23 defines an axially through engagement cavity 230, an outer surface of the protective coupler 35 forms a circumferential engagement surface 350, and the circumferential engagement surface 350 can circumferentially engage within the engagement cavity 230 to shorten an axial distance of the medical device 300 relative to the distal coupler 23.

Of course, the delivery system 100c can further include a third delivery device 40. The third delivery device 40 includes a third handle 41 and a third catheter 42 extending distally from the third handle 41. The first catheter 22 extends coaxially through the third delivery device 40. Under the manipulation of the third handle 41, the distal end of the third catheter 42 can pass through the atrial septum 51 inside the heart and be positioned in the atrium inside the heart. Under the manipulation of the first handle 21, guided by the third catheter 42, the first catheter 22 is advanced distally and extends out of the distal end of the third catheter 42 for subsequent operations. For example, the first catheter 22 has a bendable distal section 221 for performing bending adjustment operations on the distal section 221. At this time, the second catheter 32 is accommodated within the first catheter 22, the protective coupler 35 is accommodated within the distal coupler 23, and the medical device 300 engaged with the protective coupler 35 is always positioned outside the distal coupler 23.

In view of during the process of the first catheter 22 extending out of the third catheter 42, the circumferential engagement surface 350 can circumferentially engage within the engagement cavity 230, thereby shortening the axial distance by which the medical device 300 extends out of the third catheter 42, and thereby effectively avoiding the defect of atrial tissue damage caused by the medical device 300 accidentally touching the inner side of the atrial wall 52.

In order to prevent the protective coupler 35 from entering the lumen of the first catheter 22 through the engagement cavity 230 of the distal coupler 23, as shown in FIG. 18, the cross-sectional dimension of the engagement cavity 230 is greater than the cross-sectional dimension of the circumferential engagement surface 350, and the cross-sectional dimension of the circumferential engagement surface 350 is greater than the inner diameter of the first catheter 22. In at least one embodiment, the cross-section of the engagement cavity 230 and the cross-section of the circumferential engagement surface 350 are both circular; the diameter of the engagement cavity 230 is greater than the cross-sectional diameter of the circumferential engagement surface 350, and the cross-sectional diameter of the circumferential engagement surface 350 is greater than the inner diameter of the first catheter 22.

Of course, in order to better prevent the protective coupler 35 from entering the lumen of the first catheter 22 through the engagement cavity 230 of the distal coupler 23, a limiting portion 231 is disposed within the engagement cavity 230, and the limiting portion 231 is configured to limit the circumferential engagement surface 350 from entering the first catheter 22. Specifically, as shown in FIG. 19, the limiting portion 231 surrounds to form a limiting cavity 232. The cross-sectional dimension of the limiting cavity 232 is smaller than the cross-sectional dimension of the circumferential engagement surface 350, so that the circumferential engagement surface 350 is limited by the limiting cavity 232 from entering the first catheter 22. Necessarily, the cross-sectional dimension of the limiting cavity 232 is greater than the outer diameter of the second catheter 32, so that the second catheter 32 can freely pass through the limiting cavity 232 to control the protective coupler 35 to disengage from the circumferential engagement of the distal coupler 23 and extend out of the distal coupler 23. In at least one embodiment, the axial spacing S1 of the limiting portion 231 from the distal opening of the engagement cavity 230 is in the range of 0.15 mm to 2.4 mm, and the protective coupler 35 can enter or disengage from the distal coupler 23 through the distal opening of the engagement cavity 230, thereby shortening the axial distance S1. Preferably, S1 is 1.5 mm.

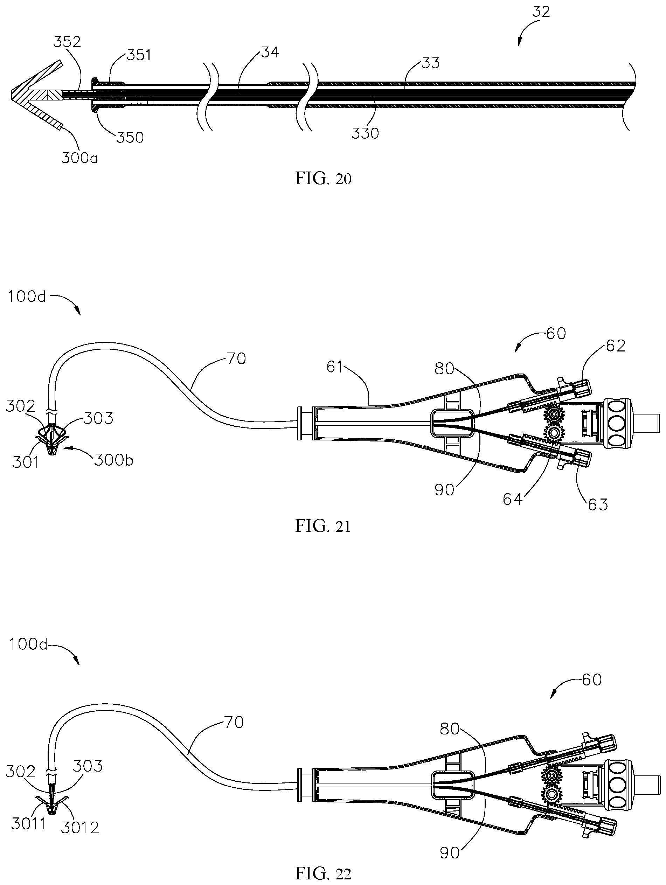

In addition, in order to ensure stable fixation of the distal coupler 23 and the protective coupler 35, the distal coupler 23 further includes a connecting portion 233 fixedly connected to the first catheter 22, and the protective coupler 35 further includes a connecting end 351 fixedly connected to the second catheter 32 (see FIG. 20). The connecting portion 233 and the connecting end 351 are both tubular, and the side walls of the tubular shape both define a plurality of openings 200 (see FIG. 17). When the distal coupler 23 is connected within the first catheter 22 by heat fusion, and the protective coupler 35 is connected within the second catheter 32 by heat fusion, the heat-fused fluid can flow into the plurality of openings 200, thereby strengthening the fixed connection between the connecting portion 233 and the connecting end 351.

In the application scenario shown in FIG. 17, after the first catheter 22 is advanced distally and extends out of the distal end of the third catheter 42, a bending adjustment operation will be further performed on the first catheter 22. Specifically, please continue to refer to FIG. 19, the first catheter 22 includes a bendable distal section 221, a proximal section 222, and a traction lumen 220 at least partially extending through the proximal section 222 and the distal section 221. A distal pull ring 223b is fixedly disposed at the distal section 221, such as heat-fused to the distal end of the distal section 221. A distal end of a traction wire 223a is attached to the distal pull ring 223b, and a proximal end of the traction wire 223a extends through the traction lumen 220 and is attached to the first handle 21. Under the manipulation of the first handle 21, the traction wire 223a can be pulled and tensioned, thereby actuating the traction wire 223a to pull the distal section 221 to bend or deform, to shape the second catheter 32 into a curve configuration having substantially the same curvature as the distal section 221 of the first catheter 22, ultimately driving the medical device 300 to move away from the inner side of the atrial wall 52, to completely avoid the inner side of the atrial wall 52.

In at least one embodiment, the distal coupler 23 is fixedly disposed at the distal end of the distal section 221, and the distal pull ring 223b is disposed within a preset range S2 from the proximal end of the distal coupler 23 in the distal section 221. Preferably, S2 is 0-2 mm.

It can be understood that, please refer to FIGS. 19 and 20 simultaneously, the second delivery device 30 further includes an axially incompressible coil 33, and the coil 33 extends coaxially through the second catheter 32. Preferably, the coil 33 is a spring tube coiled with flat wire. In at least one embodiment, the medical device 300 includes a valve clip 300a, and the protective coupler 35 further includes a protruding tubular engagement portion 352. The distal end of the tubular engagement portion 352 is releasably connected to the valve clip 300a, and the proximal end of the tubular engagement portion 352 abuts the distal end of the coil 33.

Further, the coil 33 forms an axially extending through cavity 330, a release rod 34 extends coaxially through the lumen of the coil 33 and the tubular engagement portion 352, and can switch between extending out of the distal end of the tubular engagement portion 352 and retracting to the distal end of the tubular engagement portion 352, thereby forcing the valve clip 300a to switch between being engaged with the protective coupler 35 and being disengaged with the protective coupler 35. In at least one embodiment, the cross-section of the through cavity 330 and the cross-section of the lumen of the tubular engagement portion 352 are both circular; the inner diameter of the through cavity 330 is equal to the inner diameter of the lumen of the tubular engagement portion 352, so that the release rod 34 can move smoothly within the second catheter 32.

For some applications, both the distal coupler 23 and the protective coupler 35 are made of metal materials, such as stainless steel.

In the fourth embodiment, the present disclosure further provides a valve clip system 100d with linked control, for enabling an operator to perform selectable diversified manipulation on the medical device 300, such as the valve clip 300b, satisfying the diversified needs of the operator. Specifically, please refer to FIGS. 21 and 22 simultaneously, the valve clip system 100d includes a valve clip 300b, a handle 60, a catheter 70, a first actuation element 80, and a second actuation element 90. Specifically, the valve clip 300b includes a jaw arm 301 and a first clip member 302 and a second clip member 303, where the first clip member 302 and the second clip member 303 are movable relative to the jaw arm 301. The first actuation element 80 is releasably connected to the first clip member 302, the first actuation element 80 extends proximally through the catheter 70, and is axially movable relative to the catheter 70. The second actuation element 90 is releasably connected to the second clip member 303, the second actuation element 90 extends proximally through the catheter 70, and is axially movable relative to the catheter 70. Further, the handle 60 includes a handle housing 61, a first actuation controller 62, a second actuation controller 63, and a linkage member 64. The handle housing 61 is connected to a proximal end of the catheter 70, the first actuation controller 62 is connected to a proximal end of the first actuation element 80, and the second actuation controller 63 is connected to a proximal end of the second actuation element 90. The linkage member 64 is movably coupled between the first actuation controller 62 and the second actuation controller 63, such as rotatably disposed between the first actuation controller 62 and the second actuation controller 63. The linkage member 64 is configured to move relative to the handle housing 61 to synchronously control the first actuation controller 62 and the second actuation controller 63 to move relative to the linkage member 64.

Under an external force, the linkage member 64 can decouple with the first actuation controller 62 and/or the second actuation controller 63, to cut off the linked control between the first actuation controller 62 and the second actuation controller 63, and switch to ndependent control between the first actuation controller 62 and the second actuation controller 63. The valve clip system 100d of the present disclosure can achieve linked control of the first clip member 302 and the second clip member 303 while also achieving independent control of the first clip member 302 or the second clip member 303, thereby enabling the operator to perform selectable diversified manipulation on the valve clip 300b, satisfying the diversified needs of the operator.

It can be understood that the movement of the linkage member 64 relative to the handle housing 61 can be generated by applying an external force to the first actuation controller 62 or the second actuation controller 63, or by directly applying an external force to the linkage member 64, which can be specifically referred to in the subsequent description.

For some applications, the first actuation element 80 and the second actuation element 90 are respectively wires or lines. One free end of the first actuation element 80 passes through the first clip member 302 and then folds back proximally to extend through the catheter 70, and both free ends of the first actuation element 80 are connected to the first actuation controller 62. One free end of the second actuation element 90 passes through the second clip member 303 and then folds back proximally to extend through the catheter 70, and both free ends of the second actuation element 90 are connected to the second actuation controller 63. Thus, the operator can respectively release one free end of the first actuation element 80 and the second actuation element 90, and pull the other free end of the first actuation element 80 and the second actuation element 90, to complete the release or disengagement of the first actuation element 80 at the first clip member 302, and the release or disengagement of the second actuation element 90 at the second clip member 303.

For other applications, the valve clip 300b is located outside the distal end of the catheter 70, and is releasably connected to a drive rod (release rod). The drive rod (release rod) can extend through the catheter 70 and is connected to a third actuation controller of the handle 60. Manipulating the proximal end of the third actuation controller drives the drive rod (release rod) to move relative to the catheter 70, thereby actuating the opening and closing of the distal jaw arm 301 and the disengagement of the valve clip 300b relative to the drive rod (release rod). Specifically, the jaw arm 301 is a pair of jaw arms capable of opening and closing relative to each other, including a first jaw arm 3011 and a second jaw arm 3012 hinged to the first jaw arm 3011. The first clip member 302 and the second clip member 303 are respectively elastic sheet structures. The first actuation element 80 can move axially relative to the catheter 70 under the drive of the first actuation controller 62, and is configured to actuate the first clip member 302 to move relative to the first jaw arm 3011, to switch between approaching the first jaw arm 3011 and moving away from the first jaw arm 3011, thereby completing the switching of the first clip member 302 between a clamping state clamping the heart valve and an expanded state disengaging from the heart valve. The second actuation element 90 can move axially relative to the catheter 70 under the drive of the second actuation controller 63, and is configured to actuate the second clip member 303 to move relative to the second jaw arm 3012, to switch between approaching the second jaw arm 3012 and moving away from the second jaw arm 3012, thereby completing the switching of the second clip member 303 between a clamping state clamping the heart valve and an expanded state disengaging from the heart valve.

In the fourth embodiment, the present disclosure uses the movement of the linkage member 64 to synchronously drive the first actuation controller 62 and the second actuation controller 63, thereby synchronously controlling the first clip member 302 and the second clip member 303 to clamp two leaflets of the heart valve. This linked control method is not only beneficial for the operator to manipulate the valve clip 300b, but also enables synchronous symmetrical clamping of the two leaflets of the heart valve, ensuring that the clamping depths after clamping are substantially consistent, and the surgical effect is effectively improved.

In at least one embodiment, the linkage member 64 is rotatably disposed within the handle housing 61, such as rotatably disposed within the handle housing 61, and respectively coupled to at least a distal portion of the first actuation controller 62 and at least a distal portion of the second actuation controller 63. While, the proximal end of the first actuation controller 62 and the proximal end of the second actuation controller 63 extend through the handle housing 61 and are respectively exposed outside the handle housing 61.

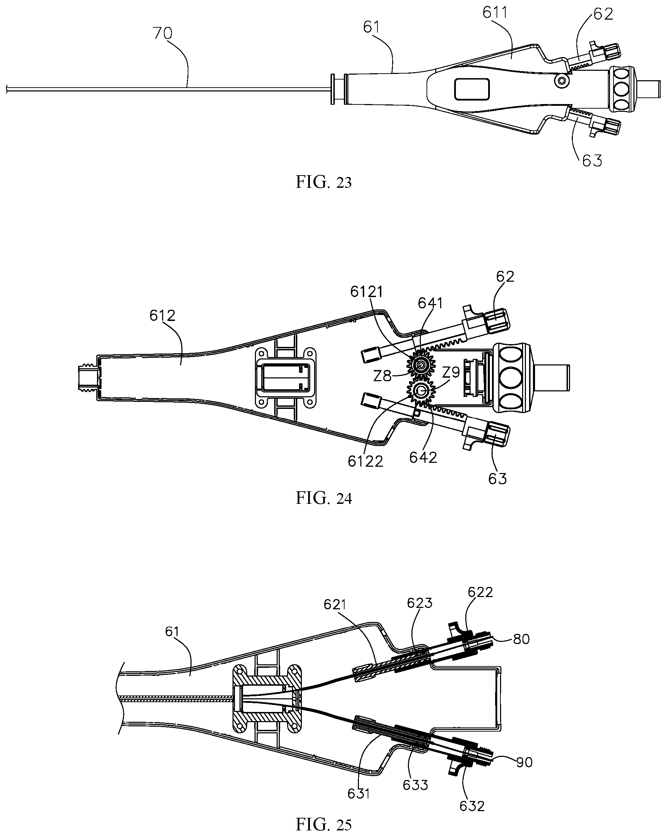

Specifically, please refer to FIG. 24, the linkage member 64 includes a first linkage member 641 and a second linkage member 642 coupled to each other. The first linkage member 641 is rotatably coupled to the first actuation controller 62, and the second linkage member 642 is rotatably coupled to the second actuation controller 63. For some applications, both the first linkage member 641 and the second linkage member 642 are configured to rotate relative to the handle housing 61. The handle housing 61 has a first radial centerline Z8 and a second radial centerline Z9 extending radially and parallel to each other. The first linkage member 641 is rotating around the first radial centerline Z8, such as rotating within the handle housing 61, and the second linkage member 642 is rotating around the second radial centerline Z9, such as rotating within the handle housing 61.

It can be understood that, please refer to FIGS. 23 and 24 simultaneously, the handle housing 61 has an upper housing 611 and a lower housing 612 cooperating with each other; the upper housing 611 and the lower housing 612 are connectable to each other to define an internal cavity. A first positioning shaft 6121 and a second positioning shaft 6122 extending from bottom to top are disposed at a proximal end of the internal cavity of the lower housing 612, for respectively positioning the first linkage member 641 and the second linkage member 642. The first positioning shaft 6121 and the second positioning shaft 6122 extend along the radial direction of the handle housing 61 and are arranged in parallel. At this time, the centerline of the first positioning shaft 6121 can be disposed as the first radial centerline Z8, and the centerline of the second positioning shaft 6122 can be disposed as the second radial centerline Z9.

In particular, the first linkage member 641 and the second linkage member 642 are gears meshing with each other. Meanwhile, at least the distal portion of the first actuation controller 62 and at least the distal portion of the second actuation controller 63 are both disposed with racks. The present disclosure preferably uses this gear-rack coupling method to achieve linked control of the valve clip system 100d, to achieve the technical effect of high transmission precision and greatly ensuring the synchronism of linked control. For example, please refer to FIGS. 21 and 22 simultaneously. Under the gear-rack transmission control, the first linkage member 641 and the second linkage member 642 will be driven to rotate in opposite directions, while the first actuation controller 62 and the second actuation controller 63 will be synchronously driven to move synchronously proximally or distally.

In order to ensure the smoothness of sliding of the first actuation controller 62 and the second actuation controller 63 at the handle housing 61, in at least one embodiment, as shown in FIG. 25, the first actuation controller 62 includes a first fixed rod 621 and a first control handle 622 slidably sleeved over the first fixed rod 621. The second actuation controller 63 includes a second fixed rod 631 and a second control handle 632 slidably sleeved over the second fixed rod 631. Further, a first seal member 623 is disposed between the first control handle 622 and the first fixed rod 621, and a second seal member 633 is disposed between the second control handle 632 and the second fixed rod 631, to ensure the sealing performance of the first actuation controller 62 and the second actuation controller 63. For example, at least one seal ring can be circumferentially disposed around the outer periphery of the proximal end of the first fixed rod 621 to close the radial gap between the first fixed rod 621 and the first control handle 622, and at least one seal ring can be circumferentially disposed around the outer periphery of the proximal end of the second fixed rod 631 to seal the radial gap between the second fixed rod 631 and the second control handle 632.

In at least one embodiment, the proximal end of the first actuation element 80 extends through the catheter 70 and the handle housing 61 (including a seal valve and PI tube located within the handle housing 61, etc.), and further extends through the first fixed rod 621 to connect within the first control handle 622, thereby ensuring that the handle 60 is reliably sealed during surgery. The proximal end of the second actuation element 90 extends through the catheter 70 and the handle housing 61 (including a seal valve and PI tube located within the handle housing 61, etc.), and further extends through the second fixed rod 631 to connect within the second control handle 632, thereby further ensuring that the handle 60 is reliably sealed during surgery.

In view of the specific structures of the first actuation controller 62 and the second actuation controller 63 being the same, the following detailed description will be given taking the specific structure of the first actuation controller 62 as an example. A first proximal rib is disposed in the internal cavity of the upper housing 611, and a second proximal rib is symmetrically disposed in the internal cavity of the lower housing 612. The first proximal rib and the second proximal rib are connected to each other to form a first sliding groove of the handle housing 61. The first fixed rod 621 is fixedly disposed at the distal end of the first sliding groove, the distal end of the first control handle 622 is sleeved outside the first fixed rod 621, and the proximal end of the first control handle 622 extends out of the handle housing 61 for the operator to manipulate. Under an external force, the first control handle 622 can slide in the first sliding groove in a direction away from or toward the first fixed rod 621.

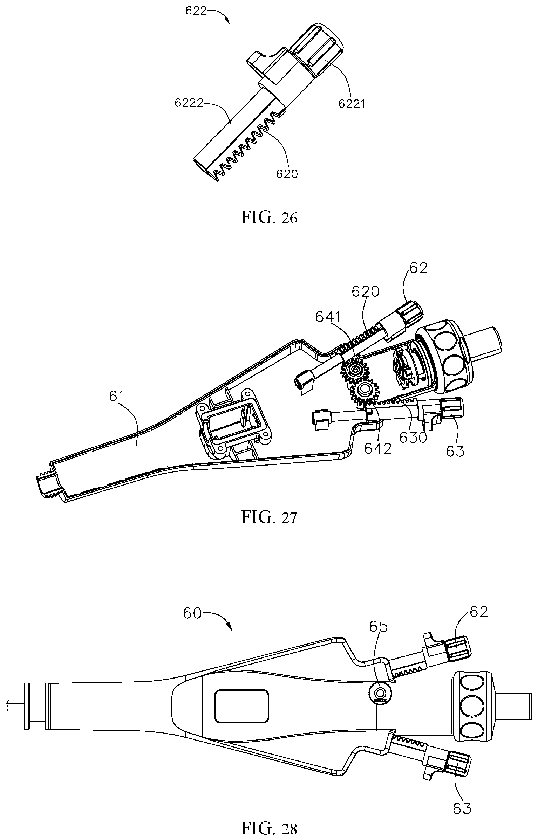

Please refer to FIG. 26 simultaneously. The first control handle 622 includes a first grip portion 6221 and a first sliding rod 6222 extending distally and protruding from the first grip portion 6221. The first grip portion 6221 is always positioned outside the handle housing 61, and at least a distal portion of the first sliding rod 6222 is sleeved outside the first fixed rod 621. Manipulating the first control handle 622 to actuate the first sliding rod 6222 to slide proximally or distally relative to the first fixed rod 621, so as to switch between extending out of the handle housing 61 and retracting into the handle housing 61. Specifically, the circumferential surface of the first grip portion 6221 is disposed with anti-slip patterns for the operator to grip conveniently. The first sliding rod 6222 is substantially a hollow rod structure, and a first rack 620 having a plurality of meshing teeth distributed axially along a side end of the first sliding rod 6222, for meshing with the meshing teeth of the first linkage member 641.

In at least one embodiment of the present disclosure, the cutting off of linked control is completed by applying an operator's actuation force to the first actuation controller 62 and/or the second actuation controller 63. As shown in FIGS. 26-27, the first actuation controller 62 can be rotationally actuated relative to the handle housing 61 to decouple such as meshing with the first linkage member 641; and/or, the second actuation controller 63 can be rotationally actuated relative to the handle housing 61 to decouple such as meshing with the second linkage member 642. This simple switching solution without additionally providing a switching knob not only makes the overall structure of the handle 60 extremely simple, but also is more conducive to convenient and easy operation.

In order to ensure the smoothness and stability when the gear and rack mesh again, only the first actuation controller 62 is rotationally actuated to disengage from meshing with the first linkage member 641. However, the first linkage member 641 and the second linkage member 642 are still in a meshed state. Then, when the second actuation controller 63 is individually pulled proximally, the first linkage member 641 can idle under the drive of the second linkage member 642 to maintain the meshed state. When linked control needs to be switched to again, the first linkage member 641 only needs to mesh with the first actuation controller 62 again, thereby increasing the controllability of the operation.

Specifically, in order to ensure that the first actuation controller 62 can smoothly rotate out of meshing with the first linkage member 641, each tooth of the first rack 620 on the first actuation controller 62 is disposed as a tooth profile feature penetrating from top to bottom. In the linked control state of the valve clip system 100d, the first rack 620 is in the meshed state with the first linkage member 641. When the first actuation controller 62 is individually controlled to rotate 90 degrees counterclockwise, the first actuation controller 62 can disengage from meshing with the first linkage member 641, thereby switching to independent control of the first actuation controller 62 and the second actuation controller 63.

Of course, in another embodiment of the present disclosure, as shown in FIGS. 28-30, the handle 60 further includes an unlocking member 65, and the unlocking member 65 is connected to the first linkage member 641 or the second linkage member 642. The unlocking member 65 is configured to shift radially along the handle housing 61 under an external force, such as shifting radially along the first radial centerline Z8 or the second radial centerline Z9. At this time, the operator can pull or push the unlocking member 65 to drive the first linkage member 641 or the second linkage member 642 to synchronously shift radially, thereby switching between disengaging from meshing with the first actuation controller 62 or the second actuation controller 63 and meshing again, thereby completing mutual switching between the linked control state and the independent control state. The following description will be given taking the unlocking member 65 connected to the first linkage member 641 as an example.

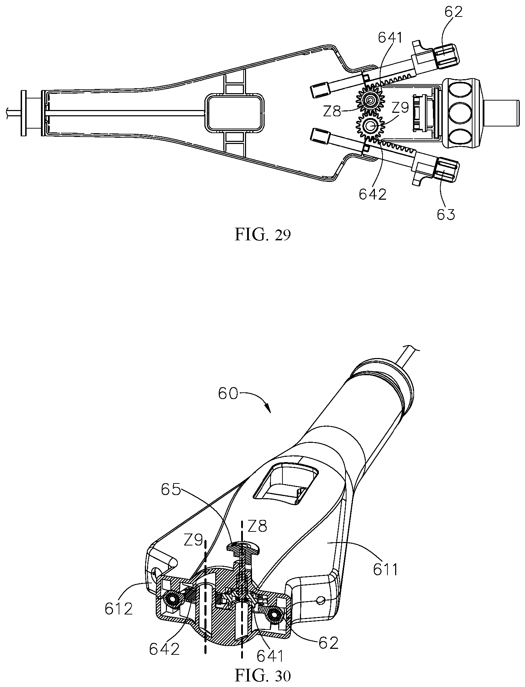

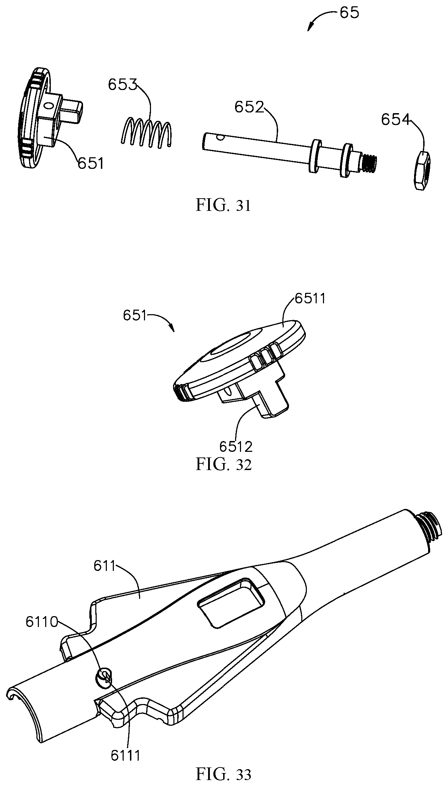

Please refer to FIGS. 30 and 31 simultaneously. The unlocking member 65 includes a knob 651, a connecting rod 652, an elastic member 653, and a limiting member 654. One end of the connecting rod 652 extends through the handle housing 61 (specifically the upper housing 611) to connect to the knob 651 located outside the handle housing 61, and the other end of the connecting rod 652 is rotatably connected to the first linkage member 641 through the limiting member 654. The elastic member 653 is sleeved outside the connecting rod 652, and is compressed between the inner surface of the handle housing 61 (specifically the inner surface of the upper housing 611) and the connecting rod 652, or between the inner surface of the handle housing 61 and the first linkage member 641.

Specifically, as shown in FIGS. 32-33, the knob 651 includes a cylindrical grip portion 6511 and a limiting boss 6512 protruding from the grip portion 6511. A circumferential surface of the cylindrical grip portion 6511 includes anti-slip patterns for the operator to grip conveniently. Correspondingly, the upper housing 611 includes a limiting portion 6110 cooperating with the knob 651. The limiting portion 6110 is substantially cylindrical and protrudes from the outer surface of the upper housing 611. The upper surface of the limiting portion 6110 recesses downward to form a limiting groove 6111, and the limiting boss 6512 can be circumferentially limited within the limiting groove 6111. Pulling the knob 651 along the direction of the first radial centerline Z8 disengages from the circumferential limitation of the limiting groove 6111.

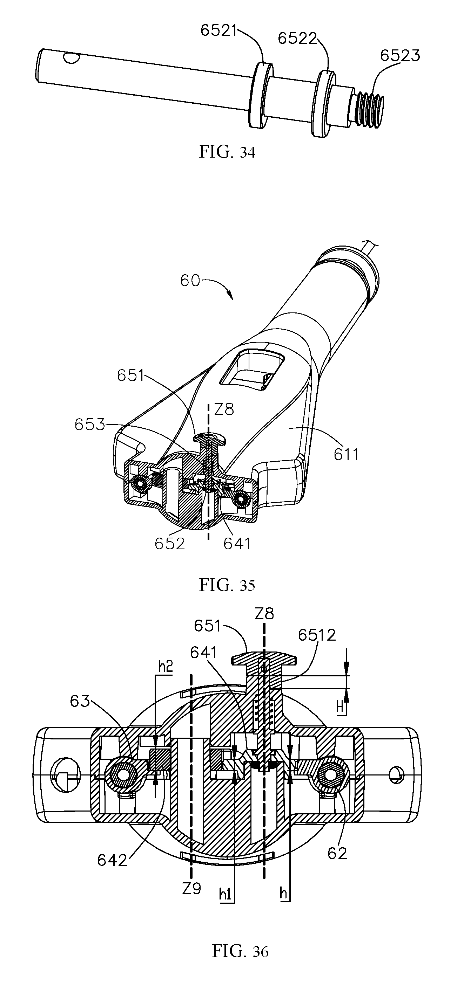

As shown in FIGS. 34-35, the connecting rod 652 is substantially an elongated rod structure, and one end of the connecting rod 652 defines a pin hole for fixed connection with the knob 651. The circumferential surface of the other end of the connecting rod 652 protrudes with a first flange 6521 and a second flange 6522. The first flange 6521 is used for abutting against the elastic member 653, and the second flange 6522 is used for abutting against the first linkage member 641. The first flange 6521 and the second flange 6522 are spaced on the circumferential side wall of the connecting rod 652 away from the knob 651, and the first flange 6521 is positioned between the second flange 6522 and the knob 651.

When the unlocking member 65 is assembled to the first linkage member 641, the end of the connecting rod 652 away from the knob 651 will be connected to the first linkage member 641 to complete coaxial assembly with the first linkage member 641. Specifically, after the connecting rod 652 passes through the central channel of the first linkage member 641, the connecting rod 652 is radially limited to the first linkage member 641 along the first radial centerline Z8 by the second flange 6522 and the limiting member 654. At this time, the second flange 6522 and the limiting member 654 will respectively abut against the radial sides of the first linkage member 641 to avoid radial shifting. That is, under the limitation of the second flange 6522 and the limiting member 654, the connecting rod 652 and the first linkage member 641 will not shift radially relative to each other, but can rotate relative to each other around the first radial centerline Z8. It should be noted that the radial direction here refers to the direction along the first radial centerline Z8. For some applications, the end of the connecting rod 652 away from the knob 651 includes external threads 6523 circumferentially, and the limiting member 654 is designed as a nut with internal threads. The unlocking member 65 uses the threaded cooperation between the external threads and the internal threads to achieve fixation of the limiting member 654 to the end of the connecting rod 652 in the first linkage member 641.

Meanwhile, the other end of the connecting rod 652 extends through a through hole of the upper housing 611 along the first radial centerline Z8 to penetrate from the internal hollow cavity to the outside, and is fixedly connected to the knob 651 outside the upper housing 611 by a pin. The through hole is interconnected with the limiting groove 6111. And, the knob 651 will be circumferentially limited to the upper housing 611 by the limiting groove 6111.

In addition, the elastic member 653 is sleeved outside the connecting rod 652 to achieve coaxial assembly with the connecting rod 652. One end of the elastic member 653 abuts against a side of the first flange 6521 away from the second flange 6522, and the other end of the elastic member 653 abuts against the inner surface of the upper housing 611. The elastic member 653 is always in a pre-compressed state to ensure that the knob 651 is always pressed tightly against the limiting portion 6110 under the elastic force of the elastic member 653 to avoid shaking. Of course, in other embodiments, the connecting rod 652 does not include the first flange 6521, and the elastic member 653 achieves its elastic connection by having one end abutting against the second flange 6522 or the first linkage member 641, and the other end abutting against the inner surface of the upper housing 611.

In order to ensure that when the unlocking member 65 is pulled radially, the first linkage member 641 can still maintain engagement with the second linkage member 642 while disengaging from the first actuation controller 62, the dimension of the first linkage member 641 along the first radial centerline Z8 may be designed to be smaller than the dimension of the second linkage member 642 along the second radial centerline Z9. Specifically, as shown in FIG. 36, the dimension (i.e., gear thickness) of the first linkage member 641 along the first radial centerline Z8 is defined as h1, and the dimension (i.e., gear thickness) of the second linkage member 642 along the second radial centerline Z9 is defined as h2. Necessarily, h2 > h1. In addition, the height at which the first linkage member 641 engages with the first actuation controller 62 is defined as h, and the height of the limiting boss 6512 of the knob 651 is defined as H. In order to ensure that after radially pulling the unlocking member 65, the limiting boss 6512 of the knob 651 can rotate to abut against the upper surface of the limiting portion 6110 to achieve stable positioning. During this stable positioning, in order to ensure that the first linkage member 641 completely disengages from the first actuation controller 62 but still maintains complete engagement with the second linkage member 642, necessarily, H > h, h2 > H.

Then, when it is necessary to switch the valve clip system 100d from the linked control state to the independent control state, the knob 651 may be pulled in the direction away from the upper housing 611 along the first radial centerline Z8 as shown in FIG. 30. Under this pulling force, the limiting boss 6512 will disengage from the restriction of the limiting groove 6111. And the first linkage member 641 will also be pulled up along the first radial centerline Z8 under the drive of the connecting rod 652, so as to disengage from engagement with the first actuation controller 62, but still maintain engagement with the second linkage member 642. At this time, the first actuation controller 62 and the second actuation controller 63 are disengaged from each other and enter the independent control state. Finally, by rotating the knob 651, the limiting boss 6512 can be brought to abut against the upper surface of the limiting portion 6110, thereby keeping the valve clip system 100d in the independent control state to facilitate subsequent manipulation by the operator.

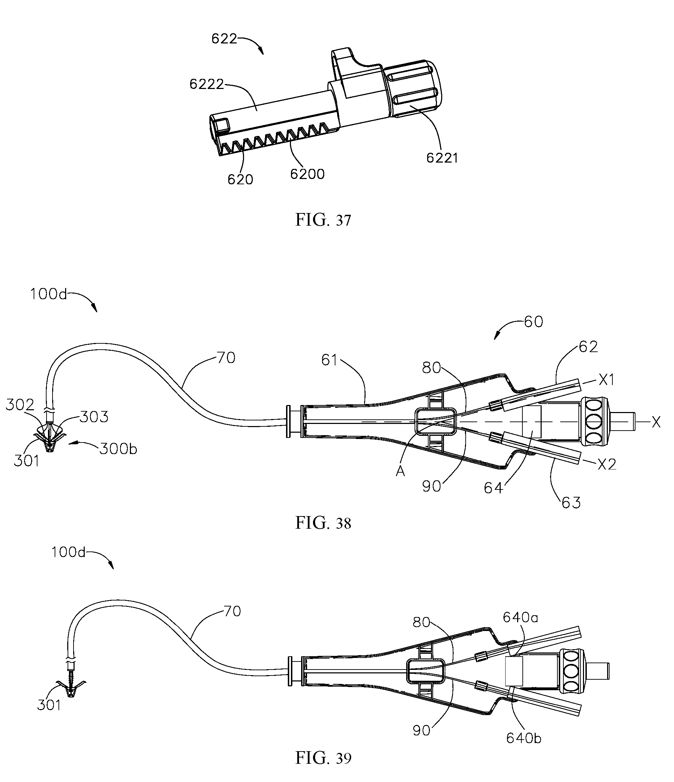

In particular, in order to avoid the occurrence of sticking during the pushing process due to tooth gaps in the rack, for some applications, as shown in FIG. 37, a bottom-closed tooth profile 6200 is disposed between each two adjacent meshing teeth of the first rack 620 of the first control handle 622, and a bottom-closed tooth profile is disposed between each two adjacent meshing teeth of the second rack of the second control handle 632, so as to retain the smooth bottom surfaces of the first sliding rod 6222 and the second sliding rod 6222. Then, during the process of pushing the first actuation controller 62 and the second actuation controller 63, the bottom-closed tooth profile design ensures the smoothness and fluency of the mating surfaces between the first control handle 622 and the second control handle 632 and the handle housing 61, thereby avoiding the occurrence of sticking.

It can be understood that for some applications, the first actuation controller 62 and the second actuation controller 63 are angularly aligned, for example, as shown in FIG. 29, the first actuation controller 62 and the second actuation controller 63 are arranged in a V-shape and located on two sides of the linkage member 64. At this time, the first actuation controller 62 and the second actuation controller 63 are arranged on opposite sides of the central axis of the handle housing 61. This distributed arrangement is not affected by the layout of the actuation controllers, which not only makes the structural space more flexible, but also prevents mutual interference and confusion that could lead to accidental touch.

In the present disclosure, in the initial state, the valve clip system 100d is in the linked control state. In this state, the first linkage member 641, which is rotatable relative to the connecting rod 652, is in an engaged state with both the second linkage member 642 and the first actuation controller 62, and the knob 651 is circumferentially limited to the upper housing 611 by the limiting groove 6111, i.e., locked to the upper housing 611 to prevent accidental touch. At this time, pulling the first actuation controller 62 or the second actuation controller 63 proximally or pushing the first actuation controller 62 or the second actuation controller 63 distally enables synchronous actuation of the first actuation element 30 and the second actuation element 40, thereby achieving linked control of the synchronous movement of the first clip member 302 and the second clip member 303 of the valve clip 300b relative to the jaw arm 301.

When it is necessary to switch the valve clip system 100d from the linked control state to the independent control state, the knob 651 is pulled and rotated so that the limiting boss 6512 of the knob 651 abuts against the upper surface of the limiting portion 6110 of the upper housing 611, thereby keeping the valve clip system 100d in the independent control state. At this time, the first linkage member 641 disengages from engagement with the first actuation controller 62, but still maintains engagement with the second linkage member 642. The first actuation controller 62 is unlocked by the first linkage member 641 and enters the independent control state, while the second actuation controller 63 still maintains engagement with the first linkage member 641 and the second linkage member 642 and enters the independent control state, with the movement of the first actuation controller 62 and the second actuation controller 63 being independent of each other. Pulling the first actuation controller 62 and the second actuation controller 63 proximally or pushing them distally enables independent actuation of the first actuation element 30 and the second actuation element 40, thereby achieving independent control of the first clip member 302 and the second clip member 303 of the valve clip 300b.

In the fifth embodiment, please also refer to FIGS. 38-39, the valve clip system 100d includes a valve clip 300b, a catheter 70, a first actuation element 80, a second actuation element 90, and a handle 60. Specifically, the valve clip 300b includes a jaw arm 301 and a first clip member 302 and a second clip member 303 movable relative to the jaw arm 301. The first actuation element 80 is releasably connected to the first clip member 302, extends proximally through the catheter 70, and is axially movable relative to the catheter 70. The second actuation element 90 is releasably connected to the second clip member 303, extends proximally through the catheter 70, and is axially movable relative to the catheter 70.

Further, the handle 60 includes a handle housing 61, a first actuation controller 62, a second actuation controller 63, and a linkage member 64. The handle housing 61 is connected to the proximal end of the catheter 70 and has a central axis X axially aligned with the catheter 70. The first actuation controller 62 is connected to the proximal end of the first actuation element 80, the second actuation controller 63 is connected to the proximal end of the second actuation element 90, and the linkage member 64 connects the first actuation controller 62 and the second actuation controller 63. The first actuation controller 62 has a first axis X1 intersecting the central axis X, and the second actuation controller 63 has a second axis X2 intersecting the central axis X. The linkage member 64 can synchronously control the first actuation controller 62 to move relative to the handle housing 61 along the first axis X1, and the second actuation controller 63 to move relative to the handle housing 61 along the second axis X2.

In at least one embodiment, the angle range between the central axis X and the first axis X1 or between the central axis X and second axis X2 is 0-90 degrees, preferably 15 degrees.

In the fifth embodiment, the first actuation controller 62 and the second actuation controller 63 are arranged at an angle to each other so as not to interfere with each other. At the same time, the is used to synchronously control the first actuation controller 62 and the second actuation controller 63, thereby synchronously controlling the first clip member 302 and the second clip member 303 to simultaneously clamp two leaflets of the heart valve, which not only meets the diverse needs of the operator, but also ensures that the clamping depths after clamping are basically consistent, thereby ensuring the surgical effect.

In order to avoid the defect that the first actuation controller 62 and the second actuation controller 63 are easily confused and accidentally touched when located on the same side of the handle housing 61, in at least one embodiment, the first axis X1 and the second axis X2 are symmetrically arranged on two sides of the central axis X. Further, in order to ensure that the first actuation controller 62 and the second actuation controller 63 can be symmetrical left and right, the extension line of the first axis X1 and the extension line of the second axis X2 intersect at point A on the central axis X (see FIG. 38).

Specifically, the linkage member 64 includes a first end 640a and a second end 640b, the first end 640a is movably connected to the first actuation controller 62, and the second end 640b is movably connected to the second actuation controller 63. The linkage member 64 is used to synchronously control the first actuation controller 62 to move proximally or distally relative to the first end 640a along the first axis X1, and the second actuation controller 63 to move proximally or distally relative to the second end 640b along the second axis X2.

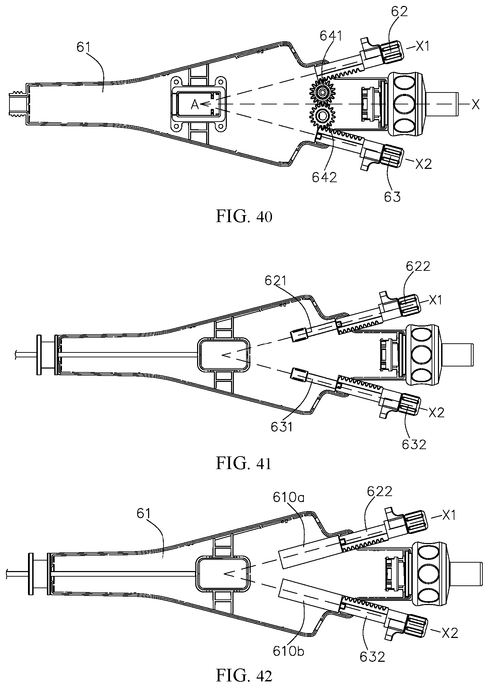

In at least one embodiment, as shown in FIG. 40, the linkage member 64 includes a first linkage member 641 and a second linkage member 642 that mesh with each other, the first linkage member 641 meshes with at least the distal portion of the first actuation controller 62, and the second linkage member 642 meshes with at least the distal portion of the second actuation controller 63. In order to ensure symmetrical engagement, the first linkage member 641 and the second linkage member 642 mesh on the central axis X. The side of the first linkage member 641 away from the second actuation controller 63 is defined as the first end 640a, and the side of the second linkage member 642 away from the first actuation controller 62 is defined as the second end 640b, for achieving meshing connection. In view of the above meshing structure and transmission mode being consistent with the fourth embodiment, they will not be described again herein.

Of course, in at least one embodiment of the present disclosure, as shown in FIG. 41, the first actuation controller 62 includes a first fixed rod 621 and a first control handle 622, and the first control handle 622 is slidably sleeved over the first fixed rod 621 along the first axis X1. The second actuation controller 63 includes a second fixed rod 631 and a second control handle 632, and the second control handle 632 is slidably sleeved over the second fixed rod 631 along the second axis X2. It can be understood that since the detailed structures of the various components of the valve clip system 100d, the connection relationships between components, and the movement relationships have all been described in the fourth embodiment, they will not be described again herein.

Of course, in other embodiments of the present disclosure, as shown in FIG. 42, the first actuation controller 62 includes only the first control handle 622 without the first fixed rod 621, and the second actuation controller 63 includes only the second control handle 632 without the second fixed rod 631. Specifically, a first guide groove 610a extending along the first axis X1 and a second guide groove 610b extending along the second axis X2 are formed at the proximal end of the handle housing 61, for the distal end of the first control handle 622 and the distal end of the second control handle 632 to slide therein respectively. For example, a first proximal rib may be disposed in the internal cavity of the upper housing 611, and a second proximal rib may be symmetrically disposed in the internal cavity of the lower housing 612, which are connected to each other to form the first guide groove 610a and the second guide groove 610b of the handle housing 61. The first axis X1 may be the central axis of the first guide groove 610a, and the second axis X2 may be the central axis of the second guide groove 610b. The first actuation controller 62 can axially move or rotate along the first axis X1 within the first guide groove 610a, and the second actuation controller 63 can axially move or rotate along the second axis X2 within the second guide groove 610b. It can be understood that since the detailed structures of other components of the valve clip system 100d, the connection relationships between components, and the movement relationships have all been described in the fourth embodiment, they will not be described again herein.

It can be understood that the delivery systems 100a, 100b, 100c, 100d can be applied to mitral valve repair, using the medical device 300 to clamp the anterior leaflet and posterior leaflet of the mitral valve, thereby avoiding mitral regurgitation. Of course, the delivery systems 100a, 100b, 100c, 100d can also be applied to tricuspid valve repair, using the medical device 300 to clamp any two adjacent leaflets of the anterior leaflet, posterior leaflet, and septal leaflet of the tricuspid valve, thereby avoiding tricuspid regurgitation. They can of course also be applied to other cardiac interventional therapeutic medical devices, which will not be described again herein.

The above descriptions are merely specific embodiments of the present application, enabling those skilled in the art to understand or implement the present disclosure. Various modifications to these embodiments will be apparent to those skilled in the art, and the general principles defined herein can be implemented in other embodiments without departing from the spirit or scope of the present application. Therefore, the present application will not be limited to the embodiments shown herein, but is to be accorded the widest scope consistent with the principles, novel features, and inventive features claimed herein.

## Claims

1. A valve clip system, comprising:
a valve clip comprising a jaw arm, a first clip member and a second clip member, wherein the first clip member and the second clip member are movable relative to the jaw arm;
a catheter;
a first actuation element and a second actuation element, wherein the first actuation element is releasably connected to the first clip member, extends proximally through the catheter and is axially movable within the catheter; the second actuation element is releasably connected to the second clip member, extends proximally through the catheter and is axially movable within the catheter; and
a handle, wherein the handle comprises:
a handle housing connected to a proximal end of the catheter;
a first actuation controller and a second actuation controller, wherein the first actuation controller is connected to a proximal end of the first actuation element, and the second actuation controller is connected to a proximal end of the second actuation element;
a first linkage member and a second linkage member, wherein the first linkage member is coupled to the first actuation controller, and the second linkage member is coupled to the second actuation controller; and
an unlocking member, wherein the unlocking member is connected to the first linkage member, the unlocking member is shiftable relative to the second linkage member under an external force, so as to switch between the first actuation controller and the second actuation controller are coupled to each other, and the coupling therebetween is released, thereby controlling the first actuation element and the second actuation element to move synchronously or independently.

2. The valve clip system according to claim 1, wherein the unlocking member is configured to drive the first linkage member to shift relative to the second linkage member, so as to drive the first linkage member to switch between coupling with the first actuation controller and decoupling with the first actuation controller, and the first linkage member and the second linkage member always maintain coupled.

3. The valve clip system according to claim 2, wherein the unlocking member drives the first linkage member to shift, so as to drive the first linkage member to decouple with the first actuation controller while maintain coupling with the second linkage member.

4. The valve clip system according to claim 3, wherein the handle housing has a first radial centerline and a second radial centerline that extend radially and are parallel to each other, the unlocking member is shiftable along the first radial centerline, and a dimension of the first linkage member along the first radial centerline is smaller than a dimension of the second linkage member along the second radial centerline.

5. The valve clip system according to any one of claims 1-4, wherein the unlocking member comprises a knob, a connecting rod, an elastic member, and a limiting member; one end of the connecting rod extends through the handle housing to connect to the knob located outside the handle housing, and the other end of the connecting rod is rotatably connected to the first linkage member through the limiting member; the elastic member is sleeved over the connecting rod and compressed between an inner surface of the handle housing and the connecting rod/the first linkage member.

6. The valve clip system according to claim 5, wherein the first linkage member has a central channel, the other end of the connecting rod is circumferentially disposed with a first flange and a second flange, the first flange is positioned between the second flange and the knob; one end of the elastic member abuts against the first flange; the other end of the connecting rod passes through the central channel and is connected to the limiting member, and the second flange and the limiting member respectively abut against two sides of the first linkage member, to rotatably connect the other end of the connecting rod to the first linkage member.

7. The valve clip system according to any one of claims 1-6, wherein the first linkage member and the second linkage member are gears meshing with each other, and at least a distal portion of the first actuation controller and at least a distal portion of the second actuation controller are each disposed with a rack.

8. A valve clip system, comprising:
a valve clip comprising a jaw arm, a first clip member and a second clip member, wherein the first clip member and the second clip member are movable relative to the jaw arm;
a catheter;
a first actuation element and a second actuation element, wherein the first actuation element is releasably connected to the first clip member, extends proximally through the catheter and is axially movable within the catheter; the second actuation element is releasably connected to the second clip member, extends proximally through the catheter and is axially movable within the catheter; and
a handle, wherein the handle comprises:
a handle housing connected to a proximal end of the catheter;
a first actuation controller and a second actuation controller, wherein the first actuation controller is connected to a proximal end of the first actuation element, and the second actuation controller is connected to a proximal end of the second actuation element; and
a first linkage member and a second linkage member, wherein the first linkage member is coupled to the first actuation controller, and the second linkage member is coupled to the second actuation controller; the first linkage member and the second linkage member are synchronously rotatable relative to the handle housing, so as to synchronously control the first actuation controller and the second actuation controller to move proximally or distally, thereby synchronously driving the first actuation element and the second actuation element to move proximally or distally.

9. The valve clip system according to claim 8, wherein the first actuation element and the second actuation element extend through the catheter in parallel, and angularly diverge at a position adjacent to the first actuation controller and the second actuation controller, and then respectively extend through the first actuation controller and the second actuation controller at an angle.

10. The valve clip system according to claim 9, wherein the first actuation element and the second actuation element respectively diverge at a 15-degrees angle and extend through the first actuation controller and the second actuation controller.

11. The valve clip system according to any one of claims 8-10, wherein the first actuation controller comprises a first control handle, and the second actuation controller comprises a second control handle; the first control handle comprises a first grip portion and a first sliding rod extending from a distal end of the first grip portion, the first grip portion is located outside the handle housing, and at least a distal portion of the first sliding rod is slidably disposed within the handle housing; the second control handle comprises a second grip portion and a second sliding rod extending from a distal end of the second grip portion, the second grip portion is located outside the handle housing, and at least a distal portion of the second sliding rod is slidably disposed within the handle housing; the first linkage member is coupled to the first sliding rod, and the second linkage member is coupled to the second sliding rod.

12. The valve clip system according to claim 11, wherein the first actuation controller further comprises a first fixed rod located within the handle housing, and the second actuation controller further comprises a second fixed rod located within the handle housing, the first sliding rod is slidably sleeved over the first fixed rod, and the second sliding rod is slidably sleeved over the second fixed rod.

13. The valve clip system according to claim 11, wherein a first rack is formed along a side end of the first sliding rod, and a second rack is formed along a side end of the second sliding rod; the first linkage member and the second linkage member are gears meshing with each other.

14. The valve clip system according to claim 13, wherein a bottom-closed tooth profile is formed between each two adjacent meshing teeth of the first rack, and a bottom-closed tooth profile is formed between each two adjacent meshing teeth of the second rack; or
a top-and-bottom through tooth profile is formed between each two adjacent meshing teeth of the first rack, and the first actuation controller is rotatably actuatable relative to the handle housing to force the first rack to disengage from meshing with the first linkage member; and/or, a top-and-bottom through tooth profile is formed between each two adjacent meshing teeth of the second rack, and the second actuation controller is rotatably actuatable relative to the handle housing to force the second rack to disengage from meshing with the second linkage member.

15. A valve clip system with linked control, comprising:
a valve clip comprising a jaw arm, a first clip member and a second clip member, wherein the first clip member and the second clip member are movable relative to the jaw arm;
a catheter;
a first actuation element and a second actuation element, wherein the first actuation element is releasably connected to the first clip member, extends proximally through the catheter and is axially movable relative to the catheter; the second actuation element is releasably connected to the second clip member, extends proximally through the catheter and is axially movable relative to the catheter; and
a handle, wherein the handle comprises:
a handle housing, wherein the handle housing is connected to a proximal end of the catheter and having a central axis axially aligned with the catheter;
a first actuation controller and a second actuation controller, wherein the first actuation controller is connected to a proximal end of the first actuation element, and the second actuation controller is connected to a proximal end of the second actuation element; and
a linkage member, wherein the linkage member is connected the first actuation controller and the second actuation controller;
the first actuation controller has a first axis intersecting the central axis, and the second actuation controller has a second axis intersecting the central axis; the linkage member is configured to synchronously control the first actuation controller to move relative to the handle housing along the first axis, and the second actuation controller to move relative to the handle housing along the second axis.

16. The valve clip system according to claim 15, wherein the linkage member is rotatably disposed within the handle housing, and is respectively coupled to at least a distal portion of the first actuation controller and at least a distal portion of the second actuation controller.

17. The valve clip system according to claim 15, wherein the linkage member comprises a first linkage member and a second linkage member meshing with each other, the first linkage member meshes with at least a distal portion of the first actuation controller, and the second linkage member meshes with at least a distal portion of the second actuation controller.

18. The valve clip system according to claim 17, wherein the first actuation controller is rotatably actuatable relative to the handle housing to decoupling with the first linkage member; and/or, the second actuation controller is rotatably actuatable relative to the handle housing to decoupling with the second linkage member.

19. The valve clip system according to claim 17, wherein the handle further comprises an unlocking member connected to the first linkage member or the second linkage member, and the unlocking member is shiftable along a radial direction of the handle housing under an external force.

20. The valve clip system according to claim 19, wherein the handle housing has a first radial centerline and a second radial centerline that extend radially and are parallel to each other, the unlocking member is connected to the first linkage member, and a dimension of the first linkage member along the first radial centerline is smaller than a dimension of the second linkage member along the second radial centerline.

21. The valve clip system according to any one of claims 17-20, wherein the first linkage member and the second linkage member mesh on the central axis.

22. The valve clip system according to claim 21, wherein the first axis and the second axis are symmetrically arranged on two sides of the central axis, or an extension line of the first axis and an extension line of the second axis intersect on the central axis.

23. The valve clip system according to claim 22, wherein an angle range between the central axis and the first axis/the second axis is 0-90 degrees.

24. The valve clip system according to any one of claims 15-23, wherein the first actuation controller comprises a first fixed rod and a first control handle, and the first control handle is slidably sleeved over the first fixed rod along the first axis; the second actuation controller comprises a second fixed rod and a second control handle, and the second control handle is slidably sleeved over the second fixed rod along the second axis.

25. A delivery system for a medical device, comprising:
a first delivery device comprising a first handle and a first catheter extending distally from the first handle, wherein the first catheter comprises a bendable distal section; and
a second delivery device comprising a second handle and a second catheter extending distally from the second handle, wherein the second catheter coaxially extends through the first delivery device, the second catheter along its axial length has a first section and a second section with a reduced outer diameter, and the second section is located distal to the first section;
wherein the first handle is manipulated to force the distal section of the first catheter to bend, the second section of the second catheter can be shapeable into a curve configuration by the bent distal section, and is at least partially recovered from the curve configuration to an initial configuration after extending out of the bent distal section.

26. The delivery system according to claim 25, wherein the second delivery device further comprises an axially incompressible coil, the coil coaxially extends through the second catheter, and the coil is configured to enhance a recovery performance of the second section from the curve configuration to the initial configuration.

27. The delivery system according to claim 25, wherein the second catheter is extendable from a distal end of the distal section and then movable with a stroke distance, and an axial length of the second section is equal to the stroke distance.

28. The delivery system according to claim 27, wherein a distal coupler is fixedly disposed at the distal end of the distal section, and the second catheter is configured to pass through the first catheter and the distal coupler in sequence and then move the stroke distance.

29. The delivery system according to claim 25, wherein a distal coupler is fixedly disposed at the distal end of the distal section, and a protective coupler is fixedly disposed at the distal end of the second section; the distal coupler has an axially through engagement cavity, and an outer surface of the protective coupler is formed with a circumferential engagement surface configured for circumferentially engaging within the engagement cavity.

30. The delivery system according to any one of claims 25-29, wherein an axial length of the second section is greater than an axial length of the distal section.

31. The delivery system according to any one of claims 25-29, wherein an outer diameter difference between the first section and the second section is in a range of 0.3 mm - 1.05 mm.

32. The delivery system according to any one of claims 25-29, wherein a smooth taper with a taper distance in a range of 10mm-25mm is disposed between the first section and the second section.

33. The delivery system according to any one of claims 25-29, wherein the second catheter comprises a lubricating coating coated on an outer surface of the second section, and a length of the lubricating coating is equal to an axial length of the second section.

34. The delivery system according to any one of claims 25-29, wherein the delivery system further comprises a third delivery device, and the third delivery device comprises a third handle and a third catheter extending distally from the third handle; the third catheter comprises a bendable section at a distal end, and the first catheter coaxially extends through the third delivery device;
wherein an outer surface of the second section and at least a portion of the first section of the second catheter has a continuous lubricating coating, and a length of the lubricating coating is greater than or equal to a sum of an axial length of the second section, an axial length of the distal section, and an axial length of the bendable section.

35. The delivery system according to any one of claims 25-29, wherein the first catheter further comprises a proximal section and a traction lumen at least partially extending through the proximal section and the distal section; at least one traction member has its distal end attached to the distal section, and its proximal end extends through the traction lumen and is attached to the first handle; the first handle is manipulated to actuate the traction member to pull the distal section, so that the distal section moves within a bending angle range of 0 degree to 90 degrees, thereby shaping the second section of the second catheter into the curve configuration having substantially the same curvature as the distal section.

36. The delivery system according to claim 35, wherein the distal section has a pre-formed curve, and the distal section is configured to move within a bending angle range of 20 degrees to 120 degrees under a combined action of the pre-formed curve and the traction member, thereby shaping the second section of the second catheter into the curve configuration having substantially the same curvature as the distal section.

37. The delivery system according to any one of claims 25-29, wherein the medical device comprises a valve clip, and the valve clip is releasably connected to the second catheter through a release rod; an inner diameter of the first section of the second catheter is equal to an inner diameter of the second section, for the release rod to pass through the first section and the second section.

38. A transcatheter delivery system for a medical device, comprising:
a first delivery device comprising a first handle and a first catheter extending distally from the first handle, wherein the first catheter comprises a bendable and/or pre-formed distal section; and
a second delivery device comprising a second handle and a second catheter extending distally from the second handle, wherein the second catheter coaxially extends through the first delivery device; and
a third delivery device comprising a third handle and a third catheter extending distally from the third handle, wherein the third catheter comprises a bendable section at a distal end, and the first catheter coaxially extends through the third delivery device;
wherein the second catheter comprises a proximal section extending along its axial length, the proximal section comprises a laser-cut tube; the second delivery device is configured to deliver the medical device through a blood vessel to a target site of a heart under a bending cooperation of the distal section and the bendable section, and the proximal section is always positioned outside a body.

39. The transcatheter delivery system according to claim 38, wherein the laser-cut tube along its axial length has a constant stiffness, and an axial length of the laser-cut tube is in a range of 400mm - 600mm.

40. The transcatheter delivery system according to claim 39, wherein the laser-cut tube has a plurality of cut patterns, the plurality of cut patterns are uniformly spaced around a circumference of the laser-cut tube; wherein a plurality of groups of the cut patterns are uniformly arranged axially on the laser-cut tube, and the cut patterns of each adjacent two groups form a radial/circumferential offset.

41. The transcatheter delivery system according to claim 40, wherein the cut patterns are one of olive-shaped patterns, waist-shaped patterns, elliptical patterns, and square patterns.

42. The transcatheter delivery system according to any one of claims 38-41, wherein a tube wall of the second catheter comprises a first polymer layer, a first reinforcement layer, and a second polymer layer from inside to outside, and the first polymer layer and the second polymer layer can bond to each other to encapsulate the first reinforcement layer between the first polymer layer and the second polymer layer; wherein the first reinforcement layer comprises the laser-cut tube and a braid mesh connected to a distal end of the laser-cut tube.

43. The transcatheter delivery system according to any one of claims 38-41, wherein the first catheter further comprises a proximal section connected to a proximal end of the distal section, the proximal section comprises a harder material than the distal section, and the harder material does not comprise a laser-cut tube; after the transcatheter delivery system delivers the medical device through the blood vessel to the target site of the heart, a portion of the proximal section is positioned outside the body, and another portion of the proximal section is positioned inside the body.

44. The transcatheter delivery system according to claim 43, wherein the first catheter further comprises a traction lumen at least partially extending through the proximal section and the distal section; at least one traction member has its distal end attached to the distal section, and its proximal end extends through a traction lumen and is attached to the first handle; the distal section has a pre-formed curve, and the distal section is configured to move within a bending angle range of 20 degrees to 120 degrees under a combined action of the pre-formed curve and the traction member.

45. A delivery system for a medical device, comprising:
a first delivery device comprising a first handle and a first catheter extending distally from the first handle, wherein a distal coupler is fixedly disposed at a distal end of the first catheter; and
a second delivery device comprising a second handle and a second catheter extending distally from the second handle, wherein the second catheter coaxially extends through the first delivery device; a protective coupler is fixedly disposed at a distal end of the second catheter, and the medical device is releasably connected to the protective coupler;
wherein the distal coupler has an axially through engagement cavity, and an outer surface of the protective coupler is formed with a circumferential engagement surface configured for circumferentially engaging within the engagement cavity, to shorten a axial distance of the medical device relative to the distal coupler.

46. The delivery system according to claim 45, wherein a cross-sectional dimension of the engagement cavity is greater than a cross-sectional dimension of the circumferential engagement surface, and the cross-sectional dimension of the circumferential engagement surface is greater than an inner diameter of the first catheter.

47. The delivery system according to claim 46, wherein a cross-section of the engagement cavity and a cross-section of the circumferential engagement surface are both circular, a diameter of the engagement cavity is greater than a diameter of the cross-sectional of the circumferential engagement surface, and the diameter of the cross-sectional of the circumferential engagement surface is greater than an inner diameter of the first catheter.

48. The delivery system according to claim 45, wherein a limiting portion is disposed within the engagement cavity, and the limiting portion is configured to limit a circumferential engagement surface from entering the first catheter.

49. The delivery system according to claim 48, wherein the limiting portion surrounds to form a limiting cavity, and a cross-sectional dimension of the limiting cavity is smaller than a cross-sectional dimension of the circumferential engagement surface, so that the circumferential engagement surface is restricted by the limiting cavity from entering the first catheter.

50. The delivery system according to claim 48, wherein an axial distance of the limiting portion from a distal opening of the engagement cavity is in a range of 0.15 mm - 2.4 mm.

51. The delivery system according to any one of claims 45-50, wherein the distal coupler further comprises a connecting portion fixedly connected to the first catheter, and the protective coupler further comprises a connecting end fixedly connected to the second catheter, both the connecting portion and the connecting end are tubular, and side walls of the tubular structures each defines a plurality of openings.

52. The delivery system according to any one of claims 45-50, wherein the first catheter comprises a bendable distal section, a proximal section, and a traction lumen at least partially extending through the proximal section and the distal section; a distal pull ring is fixedly disposed at the distal section, a distal end of a traction wire is attached to the distal pull ring, and a proximal end of the traction wire extends through the traction lumen and is attached to the first handle, the first handle is manipulated to actuate the traction wire to pull the distal section to bend; the distal coupler is fixedly disposed at the distal end of the distal section, and the distal pull ring is disposed within a preset range from the distal section to a proximal end of the distal coupler.

53. The delivery system according to any one of claims 45-50, wherein the second delivery device further comprises an axially incompressible coil, the coil coaxially extends through the second catheter; the medical device comprises a valve clip, and the protective coupler further comprises a protruding tubular engagement portion, a distal end of the tubular engagement portion is releasably connected to the valve clip, and a proximal end of the tubular engagement portion abuts a distal end of the coil.

54. The delivery system according to claim 53, wherein the coil forms an axially extending through cavity, a release rod coaxially extends through the coil and a lumen of the tubular engagement portion, and is switchable between extending from a distal end of the tubular engagement portion and retracting into the distal end of the tubular engagement portion, thereby forcing the valve clip to switch between engagement with the protective coupler and release of the engagement.
